(19) Europäisches Patentamt
European Patent Office
Office européen des brevets

(11) Veröffentlichungsnummer : **0 530 147 A1**

(12) **EUROPÄISCHE PATENTANMELDUNG**

(21) Anmeldenummer : **92810635.0**

(22) Anmeldetag : **19.08.92**

(51) Int. Cl.[5] : **C07D 207/34,** C07D 207/22, A01N 43/36, C07D 207/48

(30) Priorität : **28.08.91 CH 2516/91**

(43) Veröffentlichungstag der Anmeldung : **03.03.93 Patentblatt 93/09**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Ehrenfreund, Josef, Dr.**
**Amselstrasse 11**
**CH-4123 Allschwil (CH)**
Erfinder : **Dürr, Dieter, Dr.**
**Brändelistalweg 16**
**CH-4103 Bottmingen (CH)**

(54) **3-Cyano-2-phenyl-pyrrole als Schädlingsbekämpfungsmittel.**

(57) Verbindungen der Formel

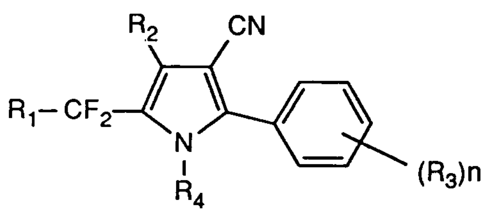

(I),

worin n die Zahl 1, 2, 3, 4 oder 5, wobei, wenn n grösser als 1 ist, die Reste $R_3$ gleich oder verschieden sind ; $R_1$ Halogen-$C_1$-$C_9$-alkyl ; $R_2$ Halogen ; $R_3$ Wasserstoff, Cyano, Nitro, Halogen, $C_1$-$C_4$-Alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen-$C_1$-$C_4$-alkyl, Halogen-$C_1$-$C_4$-alkoxy, Halogen-$C_1$-$C_4$-alkylthio , Halogen-$C_1$-$C_4$-alkansulfinyl oder Halogen-$C_1$-$C_4$-alkansulfonyl und/oder zwei an benachbarte C-Atome des Phenylrings gebundene Substituenten $R_3$ gemeinsam eine unsubstituierte oder durch Halogen, $C_1$-$C_4$-Alkyl, $C_1$-$C_4$-Alkoxy oder Halogen-$C_1$-$C_4$-alkyl substituierte Brücke, ausgewählt aus der Gruppe von Brücken, bestehend aus $-O-CH_2-O-$, $-O-CH_2-CH_2-O-$, $-O-CH_2-CH_2-$, $-O-CH_2-CH_2-CH_2-$ und -CH=CH-CH=CH- ; und $R_4$ Wasserstoff, eine unsubstituierte oder substituierte $C_1$-$C_4$-Alkylgruppe, $C_3$-$C_4$-Alkenyl, Halogen-$C_3$-$C_4$-alkenyl, $C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl oder Di-$C_1$-$C_4$-alkylaminocarbonyl bedeuten, in freier Form oder in Salzform, können als agrochemische Wirkstoffe verwendet werden und sind in an sich bekannter Weise herstellbar.

EP 0 530 147 A1

Die Erfindung betrifft Verbindungen der Formel

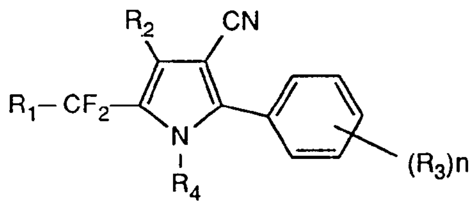

(I),

worin

n die Zahl 1, 2, 3, 4 oder 5, wobei, wenn n grösser als 1 ist, die Reste $R_3$ gleich oder verschieden sind;

$R_1$ Halogen-$C_1$-$C_9$-alkyl;

$R_2$ Halogen;

$R_3$ Wasserstoff, Cyano, Nitro, Halogen, $C_1$-$C_4$-Alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen-$C_1$-$C_4$-alkyl, Halogen-$C_1$-$C_4$-alkoxy, Halogen-$C_1$-$C_4$-alkylthio, Halogen-$C_1$-$C_4$-alkansulfinyl oder Halogen-$C_1$-$C_4$-alkansulfonyl und/oder zwei an benachbarte C-Atome des Phenylrings gebundene Substituenten $R_3$ gemeinsam eine unsubstituierte oder durch Halogen, $C_1$-$C_4$-Alkyl,
$C_1$-$C_4$-Alkoxy oder Halogen-$C_1$-$C_4$-alkyl substituierte Brücke, ausgewählt aus der Gruppe von Brücken, bestehend aus $-O-CH_2-O-$, $-O-CH_2-CH_2-O-$, $-O-CH_2-CH_2-$,
$-O-CH_2-CH_2-CH_2-$ und $-CH=CH-CH=CH-$; und

$R_4$ Wasserstoff, $C_1$-$C_4$-Alkyl, $C_3$-$C_4$-Alkenyl, Halogen-$C_3$-$C_4$-alkenyl,
$C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl, Di-$C_1$-$C_4$-alkylaminocarbonyl oder eine durch einen oder mehrere Substituenten, ausgewählt aus der Gruppe, bestehend aus Halogen, $C_1$-$C_4$-Alkoxy, Hydroxy, $C_1$-$C_4$-Alkylthio, $C_2$-$C_6$-Alkoxyalkoxy,
$C_1$-$C_4$-Alkansulfinyl, $C_1$-$C_4$-Alkansulfonyl, $C_1$-$C_4$-Alkylcarbonyl, $C_1$-$C_4$-Alkoxycarbonyl,
$C_1$-$C_4$-Alkylcarbonyloxy, Cyano, $C_2$-$C_4$-Alkenylcarbonyloxy, Phenyl, Phenoxy, Phenylthio, Benzyloxy, Benzoyloxy, Benzoyl, Phenylsulflnyl und Phenylsulfonyl, wobei die in diesen Phenyl-, Phenoxy-, Phenylthio-, Benzyloxy-, Benzoyloxy-, Benzoyl-, Phenylsulfinyl- und Phenylsulfonyl-Substituenten enthaltenen Phenylkerne jeweils durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio, $C_1$-$C_4$-Alkansulfinyl, $C_1$-$C_4$-Alkansulfonyl, Halogen-$C_1$-$C_4$-alkylthio, Halogen-$C_1$-$C_4$-alkansulfinyl, Halogen-$C_1$-$C_4$-alkansulfonyl, Cyano oder Nitro substituiert sein können, substituierte $C_1$-$C_4$-Alkylgruppe bedeuten, in freier Form oder in Salzform, ein Verfahren zur Herstellung und die Verwendung dieser Verbindungen, Schädlingsbekämpfungsmittel, deren Wirkstoff aus diesen Verbindungen, in freier Form oder in agrochemisch verwendbarer Salzform, ausgewählt ist, ein Verfahren zur Herstellung und die Verwendung dieser Mittel, mit diesen Mitteln behandeltes pflanzliches Vermehrungsgut, ein Verfahren zur Bekämpfung von Schädlingen, Zwischenprodukte, in freier Form oder in Salzform, zur Herstellung dieser Verbindungen und ein Verfahren zur Herstellung und die Verwendung dieser Zwischenprodukte.

In EP-A-0 347 488, EP-A-0 372 263, EP-A-0 426 948, EP-A-0 434 940 und EP-A-0 492 171 werden Cyanophenylpyrrol-Derivate als akarizid und insektizid wirkende Wirkstoffe in Schädlingsbekämpfungsmitteln vorgeschlagen. Die biologischen Eigenschaften der in diesen Publikationen beschriebenen Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften, insbesondere zur Bekämpfung von Insekten und Vertretern der Ordnung Akarina, zur Verfügung zu stellen, wobei diese Aufgabe erfindungsgemäss durch die Bereitstellung der vorliegenden Verbindungen I, die überraschenderweise zusätzlich auch zur Bekämpfung von phytopathogenen Pilzen geeignet sind, gelöst wird.

Verbindungen I, welche mindestens ein basisches Zentrum aufweisen, können z. B. Säureadditionssalze bilden. Diese werden beispielsweise mit starken anorganischen Säuren, wie Mineralsäuren, z. B. Perchlorsäure, Schwefelsäure, Salpetersäure, salpetrige Säure, einer Phosphorsäure oder einer Halogenwasserstoffsäure, mit starken organischen Carbonsäuren, wie gegebenenfalls, z. B. durch Halogen, substituierten $C_1$-$C_4$-Alkancarbonsäuren, z. B. Essigsäure, wie gegebenenfalls ungesättigten Dicarbonsäuren, z. B. Oxal-, Malon-, Bernstein-, Malein-, Fumar- oder Phthalsäure, wie Hydroxycarbonsäuren, z. B. Ascorbin-, Milch-, Äpfel-, Wein- oder Zitronensäure, oder wie Benzoesäure, oder mit organischen Sulfonsäuren, wie gegebenenfalls, z. B. durch Halogen, substituierten $C_1$-$C_4$-Alkan- oder Aryl-sulfonsäuren, z. B. Methan- oder p-Toluolsulfonsäure, gebildet. Ferner können Verbindungen I mit mindestens einer aciden Gruppe Salze mit Basen bilden. Geeignete Salze mit Basen sind beispielsweise Metallsalze, wie Alkali- oder Erdalkalimetallsalze, z. B. Natrium-, Kalium- oder Magnesiumsalze, oder Salze mit Ammoniak oder einem organischen Amin, wie Morpholin, Piperidin,

Pyrrolidin, einem Mono-, Di- oder Triniederalkylamin, z. B. Ethyl-, Diethyl-, Triethyl- oder Dimethyl-propylamin, oder einem Mono-, Di- oder Trihydroxyniederalkylamin,z. B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete, z. B. Bienen- oder fisch-toxische, Salze, die beispielsweise für die Isolierung bzw. Reinigung von freien Verbindungen I oder deren agrochemisch verwendbaren Salzen eingesetzt werden. Infolge der engen Beziehung zwischen den Verbindungen I in freier Form und in Form ihrer Salze sind vorstehend und nachfolgend unter den freien Verbindungen I bzw. ihren Salzen sinn- und zweckgemäss gegebenenfalls auch die entsprechenden Salze bzw. die freien Verbindungen I zu verstehen.

Die vor- und nachstehend verwendeten Allgemeinbegriffe haben, sofern nicht abweichend definiert, die nachfolgend aufgeführten Bedeutungen.

Halogen - als Gruppe per se sowie als Strukturelement von anderen Gruppen und Verbindungen, wie von Halogenalkyl, Halogenalkenyl, Halogenalkoxy, Halogenalkylthio, Halogenalkansulfinyl und Halogenalkansulfonyl, - ist Fluor, Chlor, Brom oder Iod, insbesondere Fluor, Chlor oder Brom, vor allem Fluor oder Chlor, insbesondere Fluor. Halogen $R_2$ ist vor allem Chlor oder Brom, insbesondere Brom.

Kohlenstoffhaltige Gruppen und Verbidungen enthalten, sofern nicht abweichend definiert, jeweils 1 bis und mit 9, vorzugsweise 1 bis und mit 6, bevorzugt 1 bis und mit 4, insbesondere 1 oder 2, Kohlenstoffatome.

Alkyl - als Gruppe per se sowie als Strukturelement von anderen Gruppen und Verbindungen, wie von Halogenalkyl, Alkoxy, Alkoxyalkoxy, Halogenalkoxy, Alkylthio, Halogenalkylthio, Alkansulfinyl, Halogenalkansulfinyl, Alkansulfonyl, Halogenalkansulfonyl, Dialkylaminosulfonyl, Dialkylaminocarbonyl, Alkylcarbonyl, Alkoxycarbonyl und Alkylcarbonyloxy, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl, Octyl oder Nonyl, oder verzweigt, z. B. Isopropyl, Isobutyl, sek.-Butyl, tert.-Butyl, Isopentyl, Neopentyl oder Isooctyl.

Alkenyl, Halogenalkenyl, Alkenylcarbonyloxy und Alkinyl sind geradkettig oder verzweigt und enthalten jeweils zwei oder vorzugsweise eine ungesättigte Kohlenstoff-Kohlenstoff-Bindung(en). Die Doppel- oder Dreifachbindungen dieser Substituenten sind von dem restlichen Teil der Verbindung I vorzugsweise durch mindestens ein gesättigtes Kohlenstoffatom getrennt. Beispielhaft genannt seien Allyl, Methallyl, But-2-enyl, But-3-enyl, Allylcarbonyloxy, Propargyl, But-2-inyl und But-3-inyl.

Halogen-substituierte kohlenstoffhaltige Gruppen und Verbindungen, wie Halogenalkyl, Halogenalkenyl, Halogenalkoxy, Halogenalkylthio, Halogenalkansulfinyl und Halogenalkansulfonyl, können teilweise halogeniert oder perhalogeniert sein, wobei im Falle von Mehrfach-Halogenierung die Halogensubstituenten gleich oder verschieden sein können. Beispiele für Halogenalkyl - als Gruppe per se sowie als Strukturelement von anderen Gruppen und Verbindungen, wie von Halogenalkyl, Halogenalkoxy, Halogenalkylthio, Halogenalkansulfinyl und Halogenalkansulfonyl, - sind das ein- bis dreifach durch Fluor, Chlor und/oder Brom substituierte Methyl, wie $CHF_2$ oder $CF_3$; das ein- bis fünffach durch Fluor, Chlor und/oder Brom substituierte Ethyl, wie $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl, wie $CH_2CHBrCH_2Br$, $CF_2CHFCF_3$, $CH_2CF_2CF_3$, $CF_2CF_2CF_3$ oder $CH(CF_3)_2$; und das ein- bis neunfach durch Fluor, Chlor und/oder Brom substituierte Butyl oder eines seiner Isomeren, wie $CF(CF_3)CHFCF_3$, $CF_2(CF_2)_2CF_3$ oder $CH_2(CF_2)_2CF_3$. Halogenalkyl $R_1$ ist vorzugsweise ausschliesslich fluorier, insbesondere perfluoriert. Beispiele für Halogenalkenyl sind 2-Chlorprop-1-en-3-yl, 2,3-Dichlorprop-1-en-3-yl und 2,3-Dibromprop-1-en-3-yl.

Die beiden Alkylgruppen in Dialkylaminosulfonyl- und Dialkylaminocarbonyl-Substituenten können jeweils gleich oder verschieden sein. Beispielhaft genannt seien $-SO_2-N(CH_3)_2$, $-SO_2-N(C_2H_5)_2$, $-SO_2-N(CH_3)C_2H_5$, $-SO_2-N(C_3H_7)_2$, $-SO_2-N(C_4H_9)_2$, $-CO-N(CH_3)_2$, $-CO-N(C_2H_5)_2$, $-CO-N(CH_3)C_2H_5$, $-CO-N(C_3H_7)_2$, $-CO-N(CH_3)C_3H_7$-i, $-CO-N(CH_3)C_3H_7$-n, $-CO-N(CH_3)C_4H_9$-n, $-CO-N(C_4H_9)_2$ und $-CO-N(C_2H_5)C_3H_7$-n.

In Alkoxyalkoxy ist eine an den restlichen Teil der Verbindung I gebundene Alkoxygruppe durch eine weitere Alkoxygruppe substituiert, wobei beide Kohlenstoffketten unabhängig voneinander gerade oder verzweigt sein können; Beispiele sind Methoxymethoxy, Methoxyäthoxy, Aethoxyäthoxy, Aethoxymethoxy, Propoxymethoxy, Methoxypropoxy, Butoxymethoxy und Propoxyäthoxy.

In den vorstehend aufgeführten, aus zwei Substituenten $R_3$ gemeinsam geformten, Brücken ist, wenn diese Brücken substituiert sind, entweder eines der in der unsubstituierten Grundstruktur vorhandenen Wasserstoffatome durch einen Alkyl-, Alkoxy- oder Halogenalkyl-Substituenten ersetzt oder es sind eines oder mehr als eines, insbesondere alle, der in der unsubstituierten Grundstruktur vorhandenen Wasserstoffatome durch gleiches oder verschiedenes Halogen ersetzt. Beispiele für solche substituierten Brücken sind $-O-CF_2-O-$, z. B. 2,3-$O-CF_2-O-$ oder 3,4-$O-CF_2-O-$, $-O-CF_2-CH_2-O-$, $-O-CF_2-CF_2-O-$, $-O-CHF-CHCl-$, $-O-CH-CH(CH_3)-CH_2-$, 2,3-$CH=CH-CH=CH-$, 3,4-$CH=CH-CH=CH-$, $-CH=C(OCH_3)-CH=CH-$ und $-CH=CH-C(CF_3)=CH-$.

In den substituierten Alkylgruppen $R_4$ trägt die Alkyl-Grundstruktur vorzugsweise zwei oder insbesondere

einen der vorstehend erwähnten Substituenten; handelt es sich jedoch um eine halogensubstituierte Alkylgruppe $R_4$, so sind eines oder mehr als eines, z. B. alle, der in der unsubstituierten Alkyl-Grundstruktur vorhandenen Wasserstoffatome durch gleiches oder verschiedenes Halogen ersetzt. Ist in den substituierten Alkylgruppen $R_4$ ein substituierter Phenylkern enthalten, so trägt dieser Phenylkern vorzugsweise zwei oder insbesondere einen der vorstehend erwähnten Substituenten; handelt es sich jedoch um einen halogensubstituierten Phenylkern, so können 1, 2, 3, 4 oder alle der Phenyl-Wasserstoffatome durch gleiches oder verschiedenes Halogen ersetzt sein. die Alkyl-Grundstruktur ist vorzugsweise eine Methylgruppe.

Bevorzugte Ausführungsformen im Rahmen der Erfindung sind:

(1) Eine Verbindung der Formel I, worin $R_1$ Perfluor-$C_1$-$C_6$-alkyl, insbesondere Perfluor-$C_1$-$C_4$-alkyl, vor allem $CF_3$ oder $C_2F_5$, insbesondere $CF_3$, bedeutet;

(2) Eine Verbindung der Formel I, worin $R_2$ Chlor oder Brom, insbesondere Brom, ist;

(3) Eie Verbindung der Formel I, worin n die Zahl 1, 2, 3, 4 oder 5, insbesondere 1, 2 oder 3 oder, sofern alle Reste $R_3$ gleichzeitig für Halogen stehen, 1, 2, 3, 4 oder 5, wobei, wenn n grösser als 1 ist, die Reste $R_3$ gleich oder verschieden sind, und $R_3$ Halogen, Halogen-$C_1$-$C_4$-alkyl, Halogen-$C_1$-$C_4$-alkoxy, Cyano, $C_1$-$C_4$-Alkyl, $C_1$-$C_4$-Alkoxy, $C_1$-$C_4$-Alkylthio oder $C_1$-$C_4$-Alkansulfonyl und/oder zwei an benachbarte C-Atome des Phenylrings gebundene Substituenten $R_3$ gemeinsam -O-C(Halogen)$_2$-O-, insbesondere -O-$CF_2$-O-, oder -CH=CH-CH=CH-,

insbesondere n die Zahl 1 oder 2, wobei, wenn n 2 ist, die Reste $R_3$ gleich sind, und $R_3$ Halogen, Halogen-$C_1$-$C_4$-alkyl oder Halogen-$C_1$-$C_4$-alkoxy oder zwei an benachbarte C-Atome des Phenylrings gebundene Substituenten $R_3$ gemeinsam -CH=CH-CH=CH-, vor allem n die Zahl 1 und $R_3$ Trifluormethyl, Trifluormethoxy oder Chlor, bedeuten;

(4) Eine Verbindung der Formel I, worin $R_4$ Wasserstoff, $C_1$-$C_4$-Alkyl, $C_3$-$C_4$-Alkenyl, Halogen-$C_3$-$C_4$-alkenyl, $C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl, Di-$C_1$-$C_4$-alkylaminocarbonyl oder eine durch einen oder mehrere Substituenten, ausgewählt aus der Gruppe, bestehend aus Halogen, $C_1$-$C_4$-Alkoxy, Hydroxy, $C_1$-$C_4$-Alkylthio, $C_2$-$C_6$-Alkoxyalkoxy, $C_1$-$C_4$-Alkansulfinyl, $C_1$-$C_4$-Alkansulfonyl, $C_1$-$C_4$-Alkylcarbonyl, $C_1$-$C_4$-Alkoxycarbonyl, $C_1$-$C_4$-Alkylcarbonyloxy, Cyano, $C_2$-$C_4$-Alkenylcarbonyloxy, Phenyl, Phenoxy, Phenylthio, Benzyloxy, Benzoyloxy, Benzoyl, Phenylsulfinyl und Phenylsulfonyl, wobei die in diesen Phenyl-, Phenoxy-, Phenylthio-, Benzyloxy-, Benzoyloxy-, Benzoyl-, Phenylsulfinyl- und Phenylsulfonyl-Substituenten enthaltenen Phenylkerne jeweils durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio, $C_1$-$C_4$-Alkansulfonyl, Halogen-$C_1$-$C_4$-alkylthio, Halogen-$C_1$-$C_4$-alkansulfinyl, Halogen-$C_1$ -$C_4$-alkansulfonyl, Cyano oder Nitro substituiert sein können, substituierte $C_1$-$C_4$-Alkylgruppe, insbesondere Wasserstoff, $C_1$-$C_4$-Alkyl, $C_3$-$C_4$-Alkinyl, $C_1$-$C_4$-Alkansulfonyl, Di-$C_1$-$C_4$-alkylaminosulfonyl oder eine durch $C_1$-$C_4$-Alkoxy, $C_1$-$C_4$-Alkylthio, $C_2$-$C_6$-Alkoxyalkoxy, $C_1$-$C_4$-Alkansulfonyl, $C_1$-$C_4$-Alkylcarbonyl, $C_1$-$C_4$-Alkoxycarbonyl, Cyano, Monohalogenphenyl oder Phenoxy substituierte $C_1$-$C_4$-Alkylgruppe, vor allem Wasserstoff, $C_1$-$C_4$-Alkyl oder eine durch $C_1$-$C_4$-Alkoxy oder $C_2$-$C_6$-Alkoxyalkoxy substituierte $C_1$-$C_4$-Alkylgruppe,

insbesondere Wasserstoff, $C_1$-$C_4$-Alkyl oder $C_2$-$C_6$-Alkoxyalkyl, vorzugsweise Methyl, Methoxymethyl oder Ethoxymethyl, insbesondere Wasserstoff oder Ethoxymethyl, bedeutet;

(5) Eine Verbindung der Formel I, worin $R_1$ Perfluor-$C_1$-$C_6$-alkyl, $R_2$ Chlor oder Brom, $R_3$ Halogen, Halogen-$C_1$-$C_4$-alkyl, Halogen-$C_1$-$C_4$-alkoxy, Cyano, $C_1$-$C_4$-Alkyl, $C_1$-$C_4$-Alkoxy, $C_1$-$C_4$-Alkylthio oder $C_1$-$C_4$-Alkansulfonyl, $R_4$ Wasserstoff, $C_1$-$C_4$-Alkyl oder $C_2$-$C_6$-Alkoxyalkyl und n die Zahl eins, zwei oder drei bedeuten, wobei n die Zahl eins, zwei, drei, vier oder fünf bedeuten kann, wenn alle Reste $R_3$ gleichzeitig für Halogen stehen;

(6) Eine Verbindung der Formel I, worin $R_1$ Trifluormethyl oder Pentafluorethyl, $R_2$ Brom, $R_3$ Fluor, Chlor, Trifluormethyl, Fluor-$C_1$-$C_3$-alkoxy, Methylthio, Cyano oder $C_1$-$C_3$-Alkoxy, $R_4$ Wasserstoff, Methyl, Methoxymethyl oder Ethoxymethyl und n die Zahl eins, zwei oder drei bedeuten;

(7) Eine Verbindung der Formel I, worin $R_1$ Trifluormethyl, $R_2$ Chlor oder Brom, $R_3$ Fluor, Chlor, Trifluormethyl, Fluor-$C_1$-$C_3$-alkoxy, Methylthio, Cyano oder $C_1$ -$C_3$-Alkoxy, $R_4$ Wasserstoff, Methyl, Methoxymethyl oder Ethoxymethyl und n die Zahl eins, zwei oder drei bedeuten;

(8) Eine Verbindung der Formel I, worin n die Zahl 1, $R_1$ Trifluormethyl oder Pentafluorethyl, $R_2$ Brom, $R_3$ Trifluormethyl, Trifluormethoxy oder Chlor und $R_4$ Wasserstoff oder Ethoxymethyl bedeuten.

Besonders bevorzugt sind im Rahmen der Erfindung die in den Beispielen H2, H3, H5 und H6 genannten Verbindungen der Formel I.

Namentlich bevorzugt sind im Rahmen der Erfindung

(a) 4-Brom-2-(4-chlorphenyl)-3-cyano-5-heptafluorpropyl-pyrrol,

(b) 4-Brom-2-(4-chlorphenyl)-3-cyano- 1-ethoxymethyl-5-pentafluorethyl-pyrrol,

(c) 4-Brom-2-(4-chlorphenyl)-3-cyano- 1-ethoxymethyl-5-heptafluorpropyl-pyrrol,

(d) 4-Chlor-2-(4-chlorphenyl)-3-cyano-5-heptafluorpropyl-pyrrol,
(e) 4-Chlor-2-(4-chlorphenyl)-3-cyano- 1-ethoxymethyl-5-pentafluorethyl-pyrrol,
(f) 4-Chlor-2-(4-chlorphenyl)-3-cyano- 1-ethoxymethyl-5-heptafluorpropyl-pyrrol,
(g) 4-Brom-3-cyano-5-pentafluorethyl-2-(4-trifluormethylphenyl)-pyrrol,
(h) 4-Chlor-3-cyano-5-pentafluorethyl-2-(4-trifluormethylphenyl)-pyrrol,
(i) 4-Brom-3-cyano-1-ethoxymethyl-5-pentafluorethyl-2-(4-trifluormethylphenyl)-pyrrol,
(j) 4-Chlor-3-cyano-1-ethoxymethyl-5-pentafluorethyl-2-(4-trifluormethylphenyl)-pyrrol,
(k) 4-Brom-3-cyano-5-pentafluorethyl-2-(4-trifluormethoxyphenyl)-pyrrol,
(l) 4-Chlor-3-cyano-5-pentafluorethyl-2-(4-trifluormethoxyphenyl)-pyrrol,
(m) 4-Brom-3-cyano-1-ethoxymethyl-5-pentafluorethyl-2-(4-trifluormethoxyphenyl)pyrrol und
(n) 4-Chlor-3-cyano-1-ethoxymethyl-5-pentafluorethyl-2-(4-trifluormethoxyphenyl)pyrrol.

Als weiterer Gegenstand der Erfindung ist das Verfahren zur Herstellung der Verbindungen der Formel I, in freier Form oder in Salzform, z. B. dadurch gekennzeichnet, dass man

a) in die 4-Position des Pyrrolrings einer Verbindung der Formel

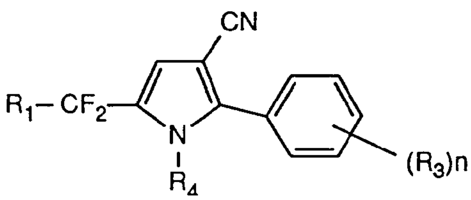

(II),

worin $R_1$, $R_3$, $R_4$ und n die für die Formel I angegebenen Bedeutungen haben, oder ein Salz davon durch Umsetzung mit einem Halogenierungsmittel, vorzugsweise in Gegenwart einer Base, den Halogensubstituenten $R_2$ einführt oder

b) zur Herstellung einer Verbindung der Formel I, worin $R_4$ von Wasserstoff verschieden ist, oder eines Salzes davon eine, z. B. gemäss der Variante a) erhältliche, Verbindung der Formel I, worin R4 Wasserstoff ist, oder ein Salz davon, vorzugsweise in Gegenwart einer Base, mit einer Verbindung der Formel

$X\text{-}R_4$ (III),

die bekannt ist oder in Analogie zu entsprechenden bekannten Verbindungen hergestellt werden kann und worin $R_4$ eine der für die Formel I angegebenen Bedeutungen mit Ausnahme von Wasserstoff hat und X für eine Abgangsgruppe steht, oder gegebenenfalls einem Salz davon umsetzt oder

c) zur Herstellung einer Verbindung der Formel I, worin $R_4$ Methyl oder eine Gruppe $R_2CH_2$- ist, oder eines Salzes davon eine Verbindung der Formel

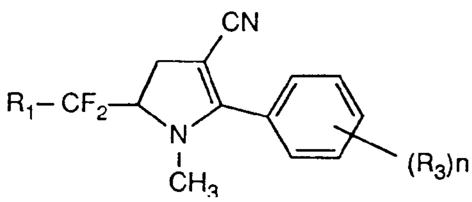

(VII),

worin $R_1$, $R_3$ und n die für die Formel I angegebenen Bedeutungen haben, oder ein Salz davon mit einem Halogenierungsmittel, gegebenenfalls in Gegenwart einer Base, gegebenenfalls in Gegenwart eines Radikalstarters, umsetzt und jeweils, wenn erwünscht, eine verfahrensgemäss oder auf andere Weise erhältliche Verbindung der Formel I, in freier Form oder in Salzform, in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

Für vor- und nachstehend aufgeführte Ausgangsmaterialien gilt im Hinblick auf deren Salze das vorstehend für Salze von Verbindungen I besagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungs-oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen I, in freier Form oder in Salzform, verwendet werden, sind bekannt oder können nach an sich bekannten Methoden, z. B. gemäss den nachstehenden Angaben, hergestellt werden.

Variante a):

Geeignete Halogenierungsmittel sind z. B. elementare Halogene, wie elementares Chlor, Brom oder Iod, Sulfurylhalogenide, wie Sulfurylchlorid oder Sulfurylbromid, oder N-Halogensuccinimide, wie N-Chlorsuccinimid oder N-Bromsuccinimid.

Geeignete Basen zur Erleichterung der Umsetzung mit dem Halogenierungsmittel sind z. B. Alkalimetall- oder Erdalkalimetall-hydroxide, -hydride, -amide, -alkanolate, -acetate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natriumhydroxid, -hydrid, -amid, -methanolat, -acetat, -carbonat, Kalium-tert.-butanolat, -hydroxid, -carbonat, -hydrid, Lithiumdiisopropylamid, Kalium-bis(trimethylsilyl)-amid, Calciumhydrid, Triethylamin, Diisopropyl-ethyl-amin, Triethylendiamin, Cyclohexylamin, N-Cyclohexyl-N,N-dimethyl-amin, N,N-Diethylanilin, Pyridin, 4-(N,N-Dimethylamino)pyridin, Chinuclidin, N-Methylmorpholin, Benzyl-trimethylammoniumhydroxid sowie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester, Ether, wie Diethylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methyl-pyrrolidon oder Hexamethylphosphorsäuretriamid; Nitrile, wie Acetonitril; Sulfoxide, wie Dimethylsulfoxid; und Säuren, z. B. starke organische Carbonsäuren, wie gegebenenfalls, z. B. durch Halogen, substituierte $C_1$-$C_4$-Alkancarbonsäuren, z. B. Ameisensäure, Essigsäure oder Propionsäure. Wird die Umsetzung in Gegenwart einer Base ausgeführt, können auch im Ueberschuss eingesetzte Basen, wie Triethylamin, pyridin, N-Methylmorpholin oder N,N-Diethylanilin, als Lösungs- oder Verdünnungsmittel dienen.

Die Umsetzung erfolgt vorteilhaft in einem Temperaturbereich von etwa 0°C bis etwa +180°C, bevorzugt von etwa +10°C bis etwa + 130°C, in vielen Fällen im Bereich zwischen der Raumtemperatur und der Rückflusstemperatur des Reaktionsgemisches. In den meisten Fällen stellt sich eine Erwärmung des Reaktionsgemisches durch freigesetzte Reaktionswärme von selbst ein. Zur Vervollständigung der Halogenierungsreaktion wird das Reaktionsgemisch vorteilhaft abschliessend noch kurze Zeit zum Rückfluss erhitzt.

Variante b):

Geeignete Abgangsgruppen X in den Verbidungen III sind z. B. Hydroxy, $C_1$-$C_8$-Alkoxy, Halogen-$C_1$-$C_8$-alkoxy, $C_1$-$C_8$-Alkanoyloxy, Mercapto, $C_1$-$C_8$-Alkylthio, Halogen-$C_1$-$C_8$-alkylthio, $C_1$-$C_8$-Alkansulfonyloxy, Halogen-$C_1$-$C_8$-alkansulfonyloxy, Benzolsulfonyloxy, Toluolsulfonyloxy und Halogen.

Geeignete Basen zur Erleichterung der HX-Abspaltung sind z. B. von der unter der Variante a) angegebenen Art.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester, Ether, wie Diethylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methyl-pyrrolidon oder Hexamethylphosphorsäuretriamid; Nitrile, wie Acetonitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa 0°C bis etwa +180°C, bevorzugt von etwa +10°C bis etwa +130°C, in vielen Fällen im Bereich zwischen der Raumtemperatur und der Rückflusstemperatur des Reaktionsgemisches.

Variante c):

Geeignete Halogenierungsmittel sind z. B. von der unter der Variante a) angegebenen Art.

Geeignete Basen zur Erleichterung der Umsetzung mit dem Halogenierungsmittel sind z. B. von der unter der Variante a) angegebenen Art.

Geeignete Radikalstarter sind z. B. Peroxo-Verbindungen, wie Dibenzoylperoxid oder Di-tert.-butylperoxid, Azo-Verbindungen, wie 2,2′-Azobisisobutyronitril, und Licht.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Solche Lösungs- oder Verdünnungsmittel sind z. B. von der unter der Variante b) angegebenen Art.

Die Umsetzung erfolgt vorteilhaft in einem Temperaturbereich von etwa 0°C bis etwa +180°C, bevorzugt von etwa +10°C bis etwa +130°C, in vielen Fällen im Bereich zwischen der Raumtemperatur und der Rückflusstemperatur des Reaktionsgemisches.

In Abhängigkeit davon, wieviele Aequivalente Halogenierungsmittel eingesetzt werden, können gemäss der Variante c) unterschiedliche Produkte der Formel I hergestellt werden. Es müssen mindestens zwei Aequivalente Halogenierungsmittel eingesetzt werden. Mittels des ersten Aequivalents wird das Dihydropyrrol-Derivat VII oxidiert und so das entsprechende Pyrrol-Zwischenprodukt ("Verbindung A") erhalten. Mittels des zweiten Aequivalents an Halogenierungsmittel wird der Halogensubstituent $R_2$ in die 4-Position des Pyrrolrings der Verbindung A eingeführt, wodurch die entsprechende Verbindung I, worin $R_4$ Methyl ist ("Verbindung B"), erhalten wird. Um die entsprechende Verbindung I, worin $R_4$ eine Gruppe $R_2CH_2$- ist, zu erhalten, d. h. um in die Methylgruppe $R_4$ der Verbindung B einen Halogensubstituenten $R_2$ einzuführen, wird ein drittes Aequivalent an Halogenierungsmittel benötigt. Der Begriff "Aequivalent" ist in diesem Absatz so zu verstehen, dass er nicht nur die genaue Menge, die exakt einem Aequivalent entspricht, sondern auch einen gewissen Bereich, z. B. etwa 10%, oberhalb und unterhalb dieser genauen Menge umfasst. Es ist möglich, die Verbindung A und/oder die Verbindung B zu isolieren, vorzugsweise wird die Reaktion aber im Sinne einer Eintopfreaktion ohne Isolierung der Verbindungen A und B durchgeführt. Weiterhin ist es möglich, die Gesamtmenge an Halogenierungsmittel zu Beginn der Umsetzung zuzusetzen, vorzugsweise wird das Halogenierungsmittel aber portionsweise zugegeben, z. B. in mehreren Portionen von jeweils einem Aequivalent. Die Monohalogenierung der Methylgruppe $R_4$ der Verbindung B wird vorzugsweise in Gegenwart eines Radikalstarters durchgeführt.

Die in der Verfahrensvariante a) als Edukte eingesetzten Verbindungen II, in freier Form oder in Salzform, sind neu und bilden ebenfalls einen Gegenstand der Erfindung. Besonders bevorzugt sind im Rahmen der Erfindung die in den Beispielen H1 und H6 genannten Verbindungen der Formel II.

Als weiterer Gegenstand der Erfindung ist das Verfahren zur Herstellung der Verbindungen der Formel II, in freier Form oder in Salzform, z. B. dadurch gekennzeichnet, dass man

d) zur Herstellung einer Verbindung der Formel II, worin $R_4$ Wasserstoff ist, oder eines Salzes davon eine Verbindung der Formel

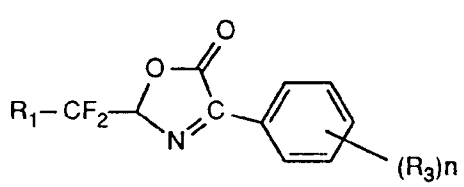

(IV),

worin $R_1$, $R_3$ und n die für die Formel I angegebenen Bedeutungen haben, oder ein Salz davon, vorzugsweise in einem hochsiedenden polaren Lösungsmittel, wie Nitromethan, und bei der Rückflusstemperatur des Reaktionsgemisches, mit 2-Chloracrylnitril umsetzt oder

e) zur Herstellung einer Verbindung der Formel II, worin $R_4$ von Wasserstoff verschieden ist, oder eines Salzes davon eine, z. B. gemäss der Variante d) erhältliche, Verbindung der Formel II, worin $R_4$ Wasser-

stoff ist, oder ein Salz davon, vorzugsweise in Gegenwart einer Base, z. B. von der unter der Variante a) angegebenen Art, mit einer Verbindung der Formel

$X-R_4$ (III),

die bekannt ist oder in Analogie zu entsprechenden bekannten Verbindungen hergestellt werden kann und worin $R_4$ eine der für die Formel I angegebenen Bedeutungen mit Ausnahme von Wasserstoff hat und X für eine Abgangsgruppe, z. B. von der unter der Variante b) angegebenen Art, steht, oder gegebenenfalls einem Salz davon umsetzt und jeweils, wenn erwünscht, eine verfahrensgemäss oder auf andere Weise erhältliche Verbindung der Formel II, in freier Form oder in Salzform, in eine andere Verbindung der Formel II überführt, ein verfahrensgemäss erhältliches Gemisch von Isomeren auftrennt und das gewünschte Isomere isoliert und/oder eine verfahrensgemäss erhältliche freie Verbindung der Formel II in ein Salz oder ein verfahrensgemäss erhältliches Salz einer Verbindung der Formel II in die freie Verbindung der Formel II oder in ein anderes Salz überführt.

Die Verbindungen IV, in freier Form oder in Salzform, sind bekannt oder können in Analogie zu bekannten Verbindungen hergestellt werden, z. B. dadurch, dass man eine Verbindung der Formel

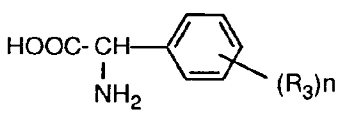

(V),

die bekannt ist oder in Analogie zu entsprechenden bekannten Verbindungen hergestellt werden kann und worin $R_3$ und n die für die Formel I angegebenen Bedeutungen haben, oder ein Salz davon mit einer Verbindung der Formel

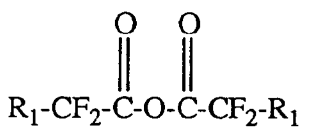

(VI),

die bekannt ist oder in Analogie zu entsprechenden bekannten Verbindungen hergestellt werden kann und worin $R_1$ die für die Formel I angegebene Bedeutung hat, umsetzt, wobei vorzugsweise bei Normaldruck, gegebenenfalls in Gegenwart eines Lösungs- oder Verdünnungsmittels, z. B. von der unter der Variante a) angegebenen Art, insbesondere in Gegenwart eines hochsiedenden aromatischen Kohlenwasserstoffs, wie Toluol, Xylol, Mesitylen oder Tetralin, und bei Temperaturen zwischen 70°C und 200°C, insbesondere zwischen 120°C und 180°C, bevorzugt bei der Siedetemperatur des Reaktionsgemisches, gearbeitet wird.

Die in der Verfahrensvariante c) als Edukte eingesetzten Verbindungen VII, in freier Form oder in Salzform, sind neu und bilden ebenfalls einen Gegenstand der Erfindung.

Als weiterer Gegenstand der Erfindung ist das Verfahren zur Herstellung der Verbindungen der Formel VII, in freier Form oder in Salzform, z. B. dadurch gekennzeichnet, dass man

f) eine Verbindung der Formel

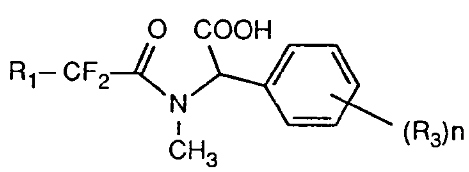

(VIII),

worin $R_1$, $R_3$ und n die für die Formel I angegebenen Bedeutungen haben, oder ein Salz davon, vorzugsweise in einem hochsiedenden polaren Lösungsmittel, wie Acetonitril, in Gegenwart eines Säureanhydrids, wie in Gegenwart von Acetanhydrid, und bei der Rückflusstemperatur des Reaktionsgemisches, mit Acrylnitril umsetzt und, wenn erwünscht, eine verfahrensgemäss oder auf andere Weise erhältliche Verbindung der Formel VII, in freier Form oder in Salzform, in eine andere Verbindung der Formel VII überführt, ein verfahrensgemäss erhältliches Gemisch von Isomeren auftrennt und das gewünschte Isomere isoliert und/oder eine verfahrensgemäss erhältliche freie Verbindung der Formel VII in ein Salz oder ein verfahrensgemäss erhältliches Salz einer Verbindung der Formel VII in die freie Verbindung der Formel VII oder in ein anderes Salz überführt.

Die Verbindungen VIII sind bekannt oder können in Analogie zu entsprechenden bekannten Verbindungen hergestellt werden.

Eine verfahrensgemäss oder auf andere Weise erhältliche Verbindung I bzw. II bzw. VII kann in an sich bekannter Weise in eine andere Verbindung I bzw. II bzw. VII überführt werden, indem man einen oder mehrere Substituenten der Ausgangsverbindung I bzw. II bzw. VII in üblicher Weise durch (einen) andere(n) erfindungsgemässe(n) Substituenten ersetzt.

Beispielsweise können:

- Hydroxygruppen (als Substituenten von Alkylgruppen $R_4$) zu Alkoxygruppen (als Substituenten von Alkylgruppen $R_4$) alkyliert werden;
- Halogen $R_3$ in unsubstituierte Positionen des Phenylrings eingeführt werden;
- Mercaptogruppen $R_3$ und/oder $R_4$ zu Sulfinyl- oder Sulfonylgruppen $R_3$ und/oder R4 oder Sulfinylgruppen $R_3$ und/oder $R_4$ zu Sulfonylgruppen $R_3$ und/oder $R_4$ oxidiert werden;
- Methylgruppen $R_4$ zu Monohalogenmethylgruppen $R_4$ monohalogeniert werden; oder
- Monohalogenmethylgruppen $R_4$ mittels Ersatzes des Halogensubstituenten durch eine Alkoxygruppe in Monoalkoxymethylgruppen $R_4$ überführt werden.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt mehrere Substituenten durch andere erfindungsgemässe Substituenten ersetzt werden.

Salze von Verbindungen I bzw. II bzw. VII können in an sich bekannter Weise hergestellt werden. So erhält man beispielsweise Säureadditionssalze von Verbindungen I bzw. II bzw. VII durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen I bzw. II bzw. VII können in üblicher Weise in die freien Verbindungen I bzw. II bzw. VII überführt werden, Säureadditionssalze z. B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I bzw. II bzw. VII können in an sich bekannter Weise in andere Salze von Verbindungen I bzw. II bzw. VII umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z. B. mit Silberacetat, in einem geeigneten Lösungsmittel, in welchem ein sich bildendes anorganisches Salz, z. B. Silberchlorid, unlöslich ist und damit aus dem Reaktionsgemisch ausscheidet.

Je nach Verfahrensweise bzw. Reaktionsbedingungen können die Verbindungen I bzw. II bzw. VII mit salzbildenden Eigenschaften in freier Form oder in Form von Salzen erhalten werden.

Die Verbindungen I bzw. II bzw. VII, in freier Form oder in Salzform, können in Form eines der möglichen Isomeren oder als Gemisch derselben, z. B. je nach Anzahl, absoluter und relativer Konfiguration von im Molekül auftretenden asymmetrischen Kohlenstoffatomen und/oder je nach Konfiguration von im Molekül auftretenden nichtaromatischen Doppelbindungen, als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I bzw. II bzw. VII, in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie Carbonsäure, z. B. Campher-, Wein- oder Äpfelsäure, oder Sulfonsäure, z. B. Camphersulfonsäure, und Trennung des auf diese Weise erhaltenen Diastereomerengemisches, z. B. auf Grund ihrer verschiedenen Löslichkeiten durch fraktionierte Kristallisation, in die Diastereomeren, aus denen das gewünschte Enantiomere durch Einwirkung geeigneter, z. B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere oder Diastereomere, oder Isomerengemisch, z. B. Enantiomerengemisch oder Diastereomerengemisch, sofern die einzelnen Komponenten unterschiedliche biologische Wirksamkeit besitzen.

Die Verbindungen I bzw. II bzw. VII, in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen.

Die Erfindung betrifft insbesondere die in den Beispielen H1 bis H6 beschriebenen Herstellungsverfahren.

Erfindungsgemäss für die Herstellung der Verbindungen I bzw. ihrer Salze verwendete Ausgangsstoffe und Zwischenprodukte, jeweils in freier Form oder in Salzform, die neu sind, ein Verfahren zu ihrer Herstellung und ihre Verwendung als Ausgangsstoffe und Zwischenprodukte für die Herstellung der Verbindungen I sind ebenfalls Gegenstand der Erfindung; insbesondere betrifft dies die Verbindungen II und VII.

Die erfindungsgemässen Verbindungen I sind auf dem Gebiet der Schädlingsbekämpfung bei günstiger Warmblüter-, Fisch- und Pflanzenverträglichkeit bereits bei niedrigen Anwendungskonzentrationen präventiv und/oder kurativ wertvolle Wirkstoffe mit einem sehr günstigen bioziden Spektrum. Die erfindungsgemässen Wirkstoffe sind gegen alle oder einzelne Entwicklungsstadien von normal sensiblen, aber auch von resistenten, tierischen Schädlingen, wie Insekten und Vertetern der Ordnung Akarina, und phytopathogenen Pilzen wirksam. Die insektizide und/oder akarizide Wirkung der erfindungsgemässen Wirkstoffe kann sich dabei direkt, d. h. in einer Abtötung der Schädlinge, welche unmittelbar oder erst nach einiger Zeit, beispielsweise bei einer Häutung, eintritt, oder indirekt, z. B. in einer verminderten Eiablage und/oder Schlupfrate, zeigen, wobei die gute Wirkung einer Abtötungsrate (Mortalität) von mindestens 50 bis 60% entspricht.

Zu den erwähnten tierischen Schädlingen gehören beispielsweise:

aus der Ordnung Lepidoptera zum Beispiel Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Y-ponomeuta spp.;

aus der Ordnung Coleoptera zum Beispiel Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;

aus der Ordnung Orthoptera zum Beispiel
Blatta spp., Blattella spp., Gryllotalpa spp., Leucophaea maderae, Locusta spp.,
Periplaneta spp. und Schistocerca spp.;

aus der Ordnung Isoptera zum Beispiel
Reticulitermes spp.;

aus der Ordnung Anoplura zum Beispiel
Liposcelis spp.;

aus der Ordnung Anoplura zum Beispiel
Haematopinus spp., Linognathus spp., Pediculus spp., Pemphigus spp. und Phylloxera spp.;

aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium comi, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis;
aus der Ordnung Thysanura zum Beispiel
Lepisma saccharina und
aus der Ordnung Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Zu den erwähnten phytopathogenen Pilzen gehören beispielsweise:
aus der Klasse der Fungi imperfecti zum Beispiel
Botrytis spp., Pyricularia spp., Helminthosporium spp., Fusarium spp., Septoria spp., Cercospora spp. und Alternaria spp.;
aus der Klasse der Basidiomyceten zum Beispiel
Rhizoctonia spp., Hemileia spp. und Puccinia spp.;
aus der Klasse der Ascomyceten zum Beispiel
Venturia spp., Erysiphe spp., Podosphaera spp., Monilinia spp. und Uncinula spp.; und
aus der Klasse der Oomyceten zum Beispiel
Phytophthora spp., Pythium spp. und Plasmopara spp..

Mit den erfindungsgemässen Wirkstoffen kann man insbesondere an Pflanzen, vor allem an Nutz- und Zierpflanzen in der Landwirtschaft, im Gartenbau und im Forst, oder an Teilen, wie Früchten, Blüten, Laubwerk, Stengeln, Knollen oder Wurzeln, solcher Pflanzen auftretende Schädlinge des erwähnten Typus bekämpfen, d. h. eindämmen oder vernichten, wobei zum Teil auch später zuwachsende Pflanzenteile noch gegen diese Schädlinge geschützt werden.

Als Zielkulturen kommen insbesondere Getreide, wie Weizen, Gerste, Roggen, Hafer, Reis, Mais oder Sorghum; Rüben, wie Zucker- oder Futterrüben; Obst, z. B. Kern-, Stein- und Beerenobst, wie Aepfel, Birnen, Pflaumen, Pfirsiche, Mandeln, Kirschen oder Beeren, z. B. Erdbeeren, Himbeeren oder Brombeeren; Hülsenfrüchte, wie Bohnen, Linsen, Erbsen oder Soja; Oelfrüchte, wie Raps, Senf, Mohn, Oliven, Sonnenblumen, Kokos, Rizinus, Kakao oder Erdnüsse; Gurkengewächse, wie Kürbisse, Gurken oder Melonen; Fasergewächse, wie Baumwolle, Flachs, Hanf oder Jute; Citrusfrüchte, wie Orangen, Zitronen, Pampelmusen oder Mandarinen; Gemüse, wie Spinat, Kopfsalat, Spargel, Kohlarten, Möhren, Zwiebeln, Tomaten, Kartoffeln oder Paprika; Lorbeergewächse, wie Avocado, Cinnamonium oder Kampfer, sowie Tabak, Nüsse, Kaffee, Eierfrüchte, Zuckerrohr, Tee, Pfeffer, Weinreben, Hopfen, Bananengewächse, Naturkautschukgewächse und Zierpflanzen, in Betracht.

Die erfindungsgemässen Wirkstoffe eignen sich besonders zur Bekämpfung von Insekten und Vertetern der Ordnung Akarina, insbesondere von pflanzenschädigenden fressenden Insekten, wie Anthonomus grandis, Diabrotica balteata, Heliothis virescens-Larven, Plutella xylostella und Spodoptera littoralis-Larven, und Spinnmilben, wie Tetranychus spp., in Baumwoll-, Obst-, Mais-, Soja-, Raps- und Gemüse-Kulturen.

Weitere Anwendungsgebiete der erfindungsgemässen Wirkstoffe sind der Schutz von Vorräten und Lagern und von Material sowie im Hygienesektor insbesondere der Schutz von Haus- und Nutztieren vor Schädlingen des erwähnten Typus.

Die Erfindung betrifft daher auch Schädlingsbekämpfungsmittel, wie, je nach angestrebten Zielen und gegebenen Verhältnissen zu wählende, emulgierbare Konzentrate, Suspensionskonzentrate, direkt versprüh- oder verdünnbare Lösungen, streichfähige Pasten, verdünnte Emulsionen, Spritzpulver, lösliche Pulver, dispergierbare Pulver, benetzbare Pulver, Stäubemittel, Granulate oder Verkapselungen in polymeren Stoffen, welche - mindestens - einen der erfindungsgemässen Wirkstoffe enthalten.

Der Wirkstoff wird in diesen Mitteln in reiner Form, ein fester Wirkstoff z. B. in einer speziellen Korngrösse, oder vorzugsweise zusammen mit - mindestens - einem der in der Formulierungstechnik üblichen Hilfsstoffe, wie Streckmitteln, z. B. Lösungsmitteln oder festen Trägerstoffen, oder wie obeflächenaktiven Verbindungen (Tensiden), eingesetzt.

Als Lösungsmittel können z. B. in Frage kommen: gegebenenfalls partiell hydrierte aromatische Kohlenwasserstoffe, bevorzugt die Fraktionen $C_8$ bis $C_{12}$ von Alkylbenzolen, wie Xylolgemische, alkylierte Naphthaline oder Tetrahydronaphthalin, aliphatische oder cycloaliphatische Kohlenwasserstoffe, wie Paraffine oder Cyclohexan, Alkohole, wie Ethanol, Propanol oder Butanol, Glykole sowie deren Ether und Ester, wie Propylenglykol, Dipropylenglykolether, Ethylenglykol oder Ethylenglykolmono-methyl- oder -ethyl-ether, Ketone, wie Cyclohexanon, Isophoron oder Diacetonalkohol, stark polare Lösungsmittel, wie N-Methylpyrrolid-2-on, Dimethylsulfoxid oder N,N-Dimethylformamid, Wasser, gegebenenfalls epoxidierte Pflanzenöle, wie gegebenenfalls epoxidiertes Raps-, Rizinus-, Kokosnuss- oder Sojaöl, und Silikonöle.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, und als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen, je nach Art des zu formulierenden Wirkstoffs, nichtionische, kationische und/oder anionische Tenside oder Tensidgemische mit guten Emulgier-, Dispergier- und Netzeigenschaften in Betracht. Die nachstehend aufgeführten Tenside sind dabei nur als Beispiele anzusehen; in der einschlägigen Literatur werden viele weitere in der Formulierungstechnik gebräuchliche und erfindungsgemäss geeignete Tenside beschrieben.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind wasserlösliche, 20 bis 250 Ethylenglykolether- und 10 bis 100 Propylenglykolether-gruppen enthaltende, Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten. Als Beispiele seien Nonylphenolpolyethoxyethanole, Ricinusölpolyglykolether, Polypropylen-Polyethylenoxid-Addukte, Tributylphenoxypolyethoxyethanol, Polyethylenglykol und Octylphenoxypolyethoxyethanol erwähnt. Ferner kommen Fettsäureester von Polyoxyethylensorbitan, wie das Polyoxyethylensorbitan-trioleat, in Betracht.

Bei den kationischen Tensiden handelt es sich vor allem um quarternäre Ammoniumsalze, welche als Substituenten mindestens einen Alkylrest mit 8 bis 22 C-Atomen und als weitere Substituenten niedrige, gegebenenfalls halogenierte, Alkyl-, Benzyl- oder niedrige Hydroxyalkylreste aufweisen. Die Salze liegen vorzugsweise als Halogenide, Methylsulfate oder Ethylsulfate vor. Beispiele sind das Stearyl-trimethyl-ammoniumchlorid und das Benzyl-di-(2-chlorethyl)-ethyl-ammoniumbromid.

Geeignete anionische Tenside können sowohl wasserlösliche Seifen als auch wasserlösliche synthetische oberflächenaktive Verbindungen sein. Als Seifen eignen sich die Alkali-, Erdalkali- und gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können; ferner sind auch die Fettsäuremethyl-taurin-salze zu erwähnen Häufiger werden

jedoch synthetische Tenside verwendet, insbesondere Fettsulfonate, Fettsulfate, sulfonierte Benzimidazoldeçrivate oder Alkylarylsulfonate. Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8 bis 22 C-Atomen. Alkylarylsulfonate sind zum Beispiel die Natrium-, Calcium- oder Triethanolammoniumsalze der Dodecylbenzolsulfonsäure, der Dibutylnaphthalinsulfonsäure oder eines Naphthalinsulfonsäure -Formaldehyd-Kondensationsproduktes. Ferner kommen auch entsprechende Phosphate, wie Salze des Phosphorsäureesters eines p-Nonylphenol-(4-14)-Ethylenoxid-Adduktes oder Phospholipide, in Frage.

Die Mittel enthalten in der Regel 0,1 bis 99%, insbesondere 0,1 bis 95%, Wirkstoff und 1 bis 99,9%, insbesondere 5 bis 99,9%, - mindestens - eines festen oder flüssigen Hilfsstoffes, wobei in der Regel 0 bis 25%, insbesondere 0,1 bis 20%, des Mittels Tenside sein können (% bedeutet jeweils Gewichtsprozent). Während als Handelsware eher konzentrierte Mittel bevorzugt werden, verwendet der Endverbraucher in der Regel verdünnte Mittel, die wesentlich geringere Wirkstoffkonzentrationen aufweisen. Bevorzugte Mittel setzen sich insbesondere folgendermassen zusammen (% = Gewichtsprozent):

Emulgierbare Konzentrate:

| | |
|---|---|
| Wirkstoff: | 1 bis 90%, vorzugsweise 5 bis 20% |
| Tensid: | 1 bis 30%, vorzugsweise 10 bis20 % |
| Lösungsmittel: | 5 bis 98%, vorzugsweise 70 bis 85% |

Stäubemittel:

| | |
|---|---|
| Wirkstoff: | 0,1 bis 10%, vorzugsweise 0,1 bis 1% |
| fester Trägerstoff: | 99,9 bis 90%, vorzugsweise 99,9 bis 99% |

Suspensionskonzentrate:

| | |
|---|---|
| Wirkstoff: | 5 bis 75%, vorzugsweise 10 bis 50% |
| Wasser. | 94 bis 24%, vorzugsweise 88 bis 30% |
| Tensid: | 1 bis 40%, vorzugsweise 2 bis 30% |

Benetzbare Pulver:

| | |
|---|---|
| Wirkstoff: | 0,5 bis 90%, vorzugsweise 1 bis 80% |
| Tensid: | 0,5 bis 20%, vorzugsweise 1 bis 15% |
| fester Trägerstoff: | 5 bis 99%, vorzugsweise 15 bis 98% |

Granulate:

| | |
|---|---|
| Wirkstoff: | 0,5 bis 30%, vorzugsweise 3 bis 15% |
| fester Trägerstoff: | 99,5 bis 70%, vorzugsweise 97 bis 85% |

Die Wirkung der erfindungsgemässen Mittel lässt sich durch Zusatz von anderen insektiziden, akariziden und/oder fungiziden Wirkstoffen wesentlich verbreitern und an gegebene Umstände anpassen. Als Wirkstoff-Zusätze kommen dabei z. B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate. Die erfindungsgemässen Mittel können auch weitere feste oder flüssige Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Düngemittel oder andere Wirkstoffe zur Erzielung spezieller Effekte, z. B. Bakterizide, Nematizide, Molluskizide oder selektive Herbizide, enthalten.

Die erfindungsgemässen Mittel werden in bekannter Weise hergestellt, bei Abwesenheit von Hilfsstoffen z. B. durch Mahlen und/oder Sieben eines festen Wirkstoffs oder Wirkstoffgemisches, z. B. auf eine bestimmte Korngrösse, und bei Anwesenheit von mindestens einem Hilfsstoff z. B. durch inniges Vermischen und/oder

Vermahlen des Wirkstoffs oder Wirkstoffgemisches mit dem (den) Hilfsstoff(en). Diese Verfahren zur Herstellung der erfindungsgemässen Mittel und die Verwendung der Verbindungen I zur Herstellung dieser Mittel bilden ebenfalls einen Gegenstand der Erfindung.

Die Anwendungsverfahren für die Mittel, also die Verfahren zur Bekämpfung von Schädlingen des erwähnten Typus, wie, je nach angestrebten Zielen und gegebenen Verhältnissen zu wählendes, Versprühen, Vernebeln, Bestäuben, Bestreichen, Beizen, Streuen oder Giessen, und die Verwendung der Mittel zur Bekämpfung von Schädlingen des erwähnten Typus sind weitere Gegenstände der Erfindung. Typische Anwendungskonzentrationen liegen dabei zwischen 0,1 und 1000 ppm, bevorzugt zwischen 0,1 und 500 ppm, Wirkstoff. Die Aufwandmengen pro Hektar betragen im allgemeinen 1 bis 2000 g Wirkstoff pro Hektar, insbesondere 10 bis 1000 g/ha, vorzugsweise 20 bis 600 g/ha.

Ein bevorzugtes Anwendungsverfahren auf dem Gebiet des Pflanzenschutzes ist das Aufbringen auf das Blattwerk der Pflanzen (Blattapplikation), wobei sich Applikationsfrequenz und Aufwandmenge auf den Befallsdruck des jeweiligen Schädlings ausrichten lassen. Der Wirkstoff kann aber auch durch das Wurzelwerk in die Pflanzen gelangen (systemische Wirkung), indem man den Standort der Pflanzen mit einem flüssigen Mittel tränkt oder den Wirkstoff in fester Form in den Standort der Pflanzen, z. B. in den Boden, einbringt, z. B. in Form von Granulat (Bodenapplikation). Bei Wasserreiskulturen kann man solche Granulate dem überfluteten Reisfeld zudosieren.

Die erfindungsgemässen Mittel eignen sich auch für den Schutz von pflanzlichem Vermehrungsgut, z. B. Saatgut, wie Früchten, Knollen oder Körnern, oder Pflanzenstecklingen, vor Pilzinfektionen und tierischen Schädlingen. Das Vermehrungsgut kann dabei vor dem Ausbringen mit dem Mittel behandelt, Saatgut z. B. vor der Aussaat gebeizt, werden. Die erfindungsgemässen Wirkstoffe können auch auf Samenkörner aufgebracht werden (Coating), indem man die Körner entweder in einem flüssigen Mittel tränkt oder sie mit einem festen Mittel beschichtet. Das Mittel kann auch beim Ausbringen des Vermehrungsguts auf den Ort der Ausbringung, z. B. bei der Aussaat in die Saatfurche, appliziert werden. Diese Behandlungsverfahren für pflanzliches Vermehrungsgut und das so behandelte pflanzliche Vermehrungsgut sind weitere Gegenstände der Erfindung.

Die folgenden Beispiele dienen der Erläuterung der Erfindung. Sie schränken die Erfindung nicht ein. Temperaturen sind in Grad Celsius angegeben.

Herstellunpsbeispiele

Beispiel H1: 2-(4-Chlorphenyl)-3-cyano-5-pentafluorethyl-pyrrol (Tabelle 2, Verbindung Nr. 2.1).

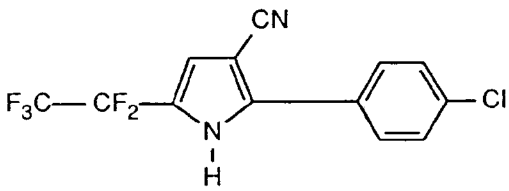

Ein Gemisch aus 47 g 4-(4-Chlorphenyl)-2,5-dihydro-5-oxo-2-pentafluorethyl-oxazol, 131,1 g 2-Chloracrylonitril und 750 ml Nitromethan wird 22 Stunden unter Rückfluss erhitzt und dann auf ca. +5° abgekühlt. Der ausgefallene Feststoff wird abfiltriert und mit wenig kaltem Dichlorethan gewaschen. Man erhält so, gewünschtenfalls unter zusätzlicher Aufarbeitung der Mutterlauge, die Titelverbindung, die bei 223,5 bis 224° schmilzt.

Beispiel H2: 4-Brom-2-(4-chlorphenyl)-3-cyano-5-pentafluorethyl-pyrrol (Tabelle 1, Verbindung Nr. 1.1).

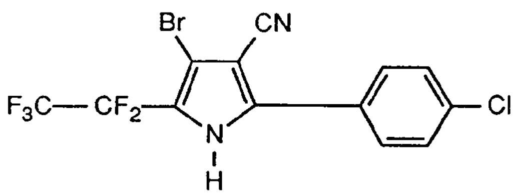

20 g 2-(4-Chlorphenyl)-3-cyano-5-pentafluorethyl-pyrrol und 12,8 g Natriumacetat werden in 400 ml Essigsäure vorgelegt. Das Gemisch wird 15 Minuten gerührt (es entsteht eine klare Lösung), innerhalb von 1 Stunde bei Raumtemperatur tropfenweise mit einer Lösung von 49,8g Brom in 100 ml Essigsäure versetzt, 2

Stunden bei Raumtemperatur gerührt, auf 21 Natriumhydrogensulfltlösung (10%) gegossen und filtriert. Die erhaltenen farblosen Kristalle werden mit Wasser gewaschen und im Vakuum bei +50° getrocknet. Man erhält so die Titelverbindung, die bei 213 bis 215° unter Zersetzung schmilzt.

Beispiel H3: 4-Brom-2-(4-chlorphenyl)-3-cyano-1-ethoxymethyl-5-pentafluorethyl-pyrrol (Tabelle 1, Verbindung Nr. 1.3).

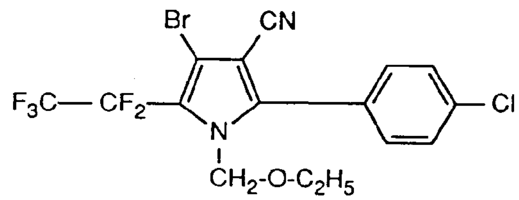

5 g 4-Brom-2- (4-chlorphenyl)-3-cyano-5-pentafluorethyl-pyrrol werden in 50 ml trockenem Tetrahydrofuran gelöst und bei Raumtemperatur mit 2,7 g Kalium-tert.-butanolat versetzt. Anschliessend lässt man 2,7 g Chlormethylethylether zutropfen. Das Reaktionsgemisch wird 22 Stunden bei Raumtemperatur gerührt, auf 400 ml Wasser gegossen und dreimal mit Diethylether extrahiert. Die organischen Phasen werden vereinigt, mit NaCl-Lösung gewaschen, über $Na_2SO_4$ getrocknet und im Vakuum eingedampft. Es resultieren gelbliche Kristalle, welche aus 10 ml Hexan umkristallisiert werden. Man erhält so die Titelverbindung, die bei 69 bis 70,5° schmilzt.

Beispiel H4: 2-(4-Chlorphenyl)-3-cyano-4,5-dihydro-1-methyl-5-pentafluorethyl-pyrrol.

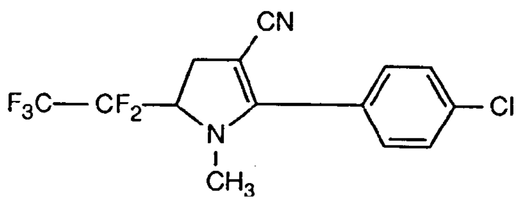

Ein Gemisch aus 15 g N-Carboxy-4-chlorphenyl-methyl-N-methyl-N-pentafluorethylcarbonyl-amin, 100 ml Acetonitril, 4.2 ml Acrylnitril, 11 g Acetanhydrid und 15 Tropfen Triethylamin wird 5.5 Stunden unter Rückfluss erhitzt und dann zur Trockne eingedampft. Reinigung des Rückstands durch Säulenchromatographie [$SiO_2$; Hexan/Ethylacetat (9:1)] liefert die Titelverbindung, die bei 113 bis 116° schmilzt.

Beispiel H5: 4-Chlor-2-(4-chlorphenyl)-3-cyano-1-methyl-5-pentafluorethyl-pyrrol (Tabelle 1, Verbindung Nr. 1.29).

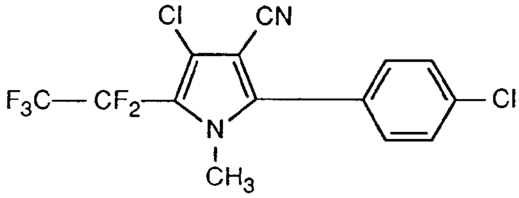

Eine gesättigte Lösung von 12 g N-Chlorsuccinimid in N,N-Dimethylformamid wird innert 30 Minuten zu einem Gemisch von 10 g 2-(4-Chlorphenyl)-3-cyano-4,5-dihydro-1 -methyl-5-pentafluorethyl-pyrrol und 10 ml N,N-Dimethylformamid zugegeben. Das Reaktionsgemisch wird 30 Minuten auf 100° erhitzt und dann in 1 l Wasser eingerührt. Der ölige Niederschlag wird in Ethylacetat aufgenommen und die Ethylacetatphase mehrmals mit Wasser gewaschen, getrocknet und zur Trockne eingedampft. Umkristallisation des Rückstands aus Ethylacetat/Hexan liefert die Titelverbindung, die bei 120° schmilzt.

Beispiel H6: In analoger Weise wie in den Beispielen H1 bis H5 beschrieben können auch die anderen in den Tabellen 1 und 2 aufgeführten Verbindungen hergestellt werden. In der Spalte "phys. Daten" dieser Tabellen bezeichnen die angegebenen Temperaturen jeweils den Schmelzpunkt der betreffenden Verbindung und "$n_D^T$" steht für den Brechungsindex der betreffenden Verbindung bei der Temperatur T°C.

Tabelle 1

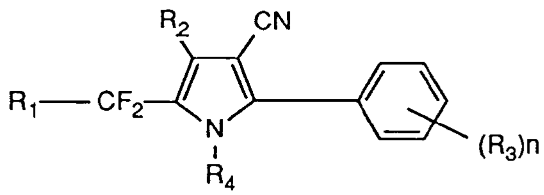

| Verb. Nr. | $R_1$ | $R_2$ | $R_3$ | n | $R_4$ | phys. Daten |
|---|---|---|---|---|---|---|
| 1.1 | $CF_3$ | Br | 4-Cl | 1 | H | 213-215° |
| 1.2 | $C_2F_5$ | Br | 4-Cl | 1 | H | 199-201° |
| 1.3 | $CF_3$ | Br | 4-Cl | 1 | $CH_2OC_2H_5$ | 69-70,5° |
| 1.4 | $C_2F_5$ | Br | 4-Cl | 1 | $CH_2OC_2H_5$ | 79-80° |
| 1.5 | $C_2F_5$ | Br | 4-F | 1 | H | 219-220,5° |
| 1.6 | $CF_3$ | Br | 4-F | 1 | $CH_2OC_2H_5$ | 85,5-86° |
| 1.7 | $C_2F_5$ | Br | 4-F | 1 | $CH_2OC_2H_5$ | 81,5-83° |
| 1.8 | $CF_3$ | Br | 4-F | 1 | H | 222,5-224° |
| 1.9 | $CF_3$ | Br | 4-Cl | 1 | $CH_3$ | 137-139° |
| 1.10 | $CF_3$ | Br | 4-$CF_3$ | 1 | H | 238-239,5° |
| 1.11 | $C_2F_5$ | Br | 4-$CF_3$ | 1 | H | 201-202° |
| 1.12 | $C_2F_5$ | Br | 4-Cl | 1 | $CH_2O(CH_2)_2OCH_3$ | 81,5-82° |
| 1.13 | $C_2F_5$ | Br | 4-Cl | 1 | $CH_3$ | 162,5-163,5° |
| 1.14 | $CF_3$ | Br | 4-$CF_3$ | 1 | $CH_2OC_2H_5$ | 86-87,5° |
| 1.15 | $C_2F_5$ | Br | 4-$CF_3$ | 1 | $CH_2OC_2H_5$ | 100,5-102,5° |
| 1.16 | $C_6F_{13}$-n | Br | 4-Cl | 1 | H | 189-190° |
| 1.17 | $C_6F_{13}$-n | Br | 4-Cl | 1 | $CH_2OC_2H_5$ | 67-69° |
| 1.18 | $CF_3$ | Br | 4-Cl | 1 | $CH_2O(CH_2)_2OCH_3$ | $n_D^{26} = 1,5228$ |
| 1.19 | $CF_3$ | Br | 2,4$(Cl)_2$ | 2 | H | 172-174° |
| 1.20 | $C_2F_5$ | Br | 2,4$(Cl)_2$ | 2 | H | 155-156° |
| 1.21 | $CF_3$ | Br | 2,4$(Cl)_2$ | 2 | $CH_2OC_2H_5$ | $n_D^{26,5} = 1,5325$ |
| 1.22 | $C_2F_5$ | Br | 2,4$(Cl)_2$ | 2 | $CH_2OC_2H_5$ | $n_D^{26,5} = 1,5134$ |
| 1.23 | $CF_3$ | Br | 3,4$(Cl)_2$ | 2 | $CH_3$ | |
| 1.24 | $CF_3$ | Br | 3,4$(Cl)_2$ | 2 | $CH_2OC_2H_5$ | 114-116° |
| 1.25 | $CF_3$ | Br | 3,4$(Cl)_2$ | 2 | $CH_2CN$ | |
| 1.26 | $CF_3$ | Br | 3,4$(Cl)_2$ | 2 | $CH_2C \equiv CH$ | |

| Verb. Nr. | $R_1$ | $R_2$ | $R_3$ | n | $R_4$ | phys. Daten |
|---|---|---|---|---|---|---|
| 1.27 | $C_2F_5$ | Br | $3,4(Cl)_2$ | 2 | $CH_3$ | |
| 1.28 | $C_2F_5$ | Br | $3,4(Cl)_2$ | 2 | $CH_2OC_2H_5$ | 107-108° |
| 1.29 | $CF_3$ | Cl | 4-Cl | 1 | $CH_3$ | 120° |
| 1.30 | $CF_3$ | Cl | 4-Cl | 1 | $CH_2OC_2H_5$ | 76,5-77° |
| 1.31 | $C_2F_5$ | Cl | 4-Cl | 1 | $CH_3$ | |
| 1.32 | $C_2F_5$ | Cl | 4-Cl | 1 | $CH_2OC_2H_5$ | 78,5-80° |
| 1.33 | $CF_3$ | Cl | $4-CF_3$ | 1 | $CH_3$ | |
| 1.34 | $CF_3$ | Cl | $4-CF_3$ | 1 | $CH_2OC_2H_5$ | |
| 1.35 | $C_2F_5$ | Cl | $4-CF_3$ | 1 | $CH_3$ | |
| 1.36 | $C_2F_5$ | Cl | $4-CF_3$ | 1 | $CH_2OC_2H_5$ | |
| 1.37 | $CF_3$ | Cl | $2,4(Cl)_2$ | 2 | $CH_3$ | |
| 1.38 | $CF_3$ | Cl | $2,4(Cl)_2$ | 2 | $CH_2OC_2H_5$ | |
| 1.39 | $C_2F_5$ | Cl | $2,4(Cl)_2$ | 2 | $CH_3$ | |
| 1.40 | $C_2F_5$ | Cl | $2,4(Cl)_2$ | 2 | $CH_2OC_2H_5$ | |
| 1.41 | $CF_3$ | Cl | $3,4(Cl)_2$ | 2 | $CH_3$ | |
| 1.42 | $CF_3$ | Cl | $3,4(Cl)_2$ | 2 | $CH_2OC_2H_5$ | |
| 1.43 | $C_2F_5$ | Cl | $3,4(Cl)_2$ | 2 | $CH_3$ | |
| 1.44 | $C_2F_5$ | Cl | $3,4(Cl)_2$ | 2 | $CH_2OC_2H_5$ | |
| 1.45 | $CF_3$ | Br | 4-Cl | 1 | $C_2H_5$ | |
| 1.46 | $CF_3$ | Br | 4-Cl | 1 | $CH_2C{\equiv}CH$ | |
| 1.47 | $CF_3$ | Br | 4-Cl | 1 | $CH_2CN$ | |
| 1.48 | $CF_3$ | Br | 4-Cl | 1 | $CH_2O-C_6H_5$ | |
| 1.49 | $CF_3$ | Br | 4-Cl | 1 | $CH_2OCH_3$ | |
| 1.50 | $CF_3$ | Br | 4-Cl | 1 | $CH_2SCH_3$ | |
| 1.51 | $CF_3$ | Br | 4-Cl | 1 | $CH_2COCH_3$ | |
| 1.52 | $CF_3$ | Br | 4-Cl | 1 | $CH_2COOCH_3$ | |
| 1.53 | $CF_3$ | Br | 4-Cl | 1 | $SO_2CH_3$ | |
| 1.54 | $CF_3$ | Br | 4-Cl | 1 | $SO_2N(CH_3)_2$ | |
| 1.55 | $CF_3$ | Br | 4-Cl | 1 | $CH_2SC_2H_5$ | |
| 1.56 | $CF_3$ | Br | 4-Cl | 1 | $CH_2SO_2C_2H_5$ | |
| 1.57 | $CF_3$ | Br | 4-Cl | 1 | $CH_2-C_6H_4-Cl(4)$ | |
| 1.58 | $C_2F_5$ | Br | 4-Cl | 1 | $C_2H_5$ | |

| Verb. Nr. | $R_1$ | $R_2$ | $R_3$ | n | $R_4$ | phys. Daten |
|---|---|---|---|---|---|---|
| 1.59 | $C_2F_5$ | Br | 4-Cl | 1 | $CH_2C{\equiv}CH$ | |
| 1.60 | $C_2F_5$ | Br | 4-Cl | 1 | $CH_2CN$ | |
| 1.61 | $C_2F_5$ | Br | 4-Cl | 1 | $CH_2O$-$C_6H_5$ | |
| 1.62 | $C_2F_5$ | Br | 4-Cl | 1 | $CH_2OCH_3$ | |
| 1.63 | $C_2F_5$ | Br | 4-Cl | 1 | $CH_2SCH_3$ | |
| 1.64 | $C_2F_5$ | Br | 4-Cl | 1 | $CH_2COCH_3$ | |
| 1.65 | $C_2F_5$ | Br | 4-Cl | 1 | $CH_2COOCH_3$ | |
| 1.66 | $C_2F_5$ | Br | 4-Cl | 1 | $SO_2CH_3$ | |
| 1.67 | $C_2F_5$ | Br | 4-Cl | 1 | $SO_2N(CH_3)_2$ | |
| 1.68 | $C_2F_5$ | Br | 4-Cl | 1 | $CH_2SC_2H_5$ | |
| 1.69 | $C_2F_5$ | Br | 4-Cl | 1 | $CH_2SO_2C_2H_5$ | |
| 1.70 | $C_2F_5$ | Br | 4-Cl | 1 | $CH_2$-$C_6H_4$-Cl(4) | |
| 1.71 | $CF_3$ | Br | 2,4,6$(Cl)_3$ | 3 | $CH_3$ | |
| 1.72 | $CF_3$ | Br | 2,4,6$(Cl)_3$ | 3 | $CH_2OC_2H_5$ | |
| 1.73 | $C_2F_5$ | Br | 2,4,6$(Cl)_3$ | 3 | $CH_3$ | |
| 1.74 | $C_2F_5$ | Br | 2,4,6$(Cl)_3$ | 3 | $CH_2OC_2H_5$ | |
| 1.75 | $CF_3$ | Br | 4-Br | 1 | $CH_3$ | |
| 1.76 | $CF_3$ | Br | 4-Br | 1 | $CH_2OC_2H_5$ | |
| 1.77 | $C_2F_5$ | Br | 4-Br | 1 | $CH_3$ | |
| 1.78 | $C_2F_5$ | Br | 4-Br | 1 | $CH_2OC_2H_5$ | |
| 1.79 | $CF_3$ | Br | 4-$OCF_3$ | 1 | $CH_3$ | |
| 1.80 | $CF_3$ | Br | 4-$OCF_3$ | 1 | $CH_2OC_2H_5$ | $n_D^{22}$=1,4872 |
| 1.81 | $C_2F_5$ | Br | 4-$OCF_3$ | 1 | $CH_3$ | |
| 1.82 | $C_2F_5$ | Br | 4-$OCF_3$ | 1 | $CH_2OC_2H_5$ | $n_D^{22,5}$= 1,4732 |
| 1.83 | $CF_3$ | Br | 4-$OCF_2CHF_2$ | 1 | $CH_3$ | |
| 1.84 | $CF_3$ | Br | 4-$OCF_2CHF_2$ | 1 | $CH_2OC_2H_5$ | |
| 1.85 | $C_2F_5$ | Br | 4-$OCF_2CHF_2$ | 1 | $CH_3$ | |
| 1.86 | $C_2F_5$ | Br | 4-$OCF_2CHF_2$ | 1 | $CH_2OC_2H_5$ | |
| 1.87 | $CF_3$ | Br | 4-$OCF_2CHFCF_3$ | 1 | $CH_3$ | |
| 1.88 | $CF_3$ | Br | 4-$OCF_2CHFCF_3$ | 1 | $CH_2OC_2H_5$ | |
| 1.89 | $C_2F_5$ | Br | 4-$OCF_2CHFCF_3$ | 1 | $CH_3$ | |

| Verb. Nr. | $R_1$ | $R_2$ | $R_3$ | n | $R_4$ | phys. Daten |
|---|---|---|---|---|---|---|
| 1.90 | $C_2F_5$ | Br | 4-$OCF_2CHFCF_3$ | 1 | $CH_2OC_2H_5$ | |
| 1.91 | $CF_3$ | Br | 2,3,4,5,6$(F)_5$ | 5 | $CH_3$ | |
| 1.92 | $CF_3$ | Br | 2,3,4,5,6$(F)_5$ | 5 | $CH_2OC_2H_5$ | |
| 1.93 | $C_2F_5$ | Br | 2,3,4,5,6$(F)_5$ | 5 | $CH_3$ | |
| 1.94 | $C_2F_5$ | Br | 2,3,4,5,6$(F)_5$ | 5 | $CH_2OC_2H_5$ | |
| 1.95 | $CF_3$ | Br | 4-$OCH_2CF_3$ | 1 | $CH_3$ | |
| 1.96 | $CF_3$ | Br | 4-$OCH_2CF_3$ | 1 | $CH_2OC_2H_5$ | |
| 1.97 | $C_2F_5$ | Br | 4-$OCH_2CF_3$ | 1 | $CH_3$ | |
| 1.98 | $C_2F_5$ | Br | 4-$OCH_2CF_3$ | 1 | $CH_2OC_2H_5$ | |
| 1.99 | $CF_3$ | Br | 2,6$(Cl)_2$,4$CF_3$ | 3 | $CH_3$ | |
| 1.100 | $CF_3$ | Br | 2,6$(Cl)_2$,4$CF_3$ | 3 | $CH_2OC_2H_5$ | |
| 1.101 | $C_2F_5$ | Br | 2,6$(Cl)_2$,4$CF_3$ | 3 | $CH_3$ | |
| 1.102 | $C_2F_5$ | Br | 2,6$(Cl)_2$,4$CF_3$ | 3 | $CH_2OC_2H_5$ | |
| 1.103 | $CF_3$ | Br | 3,4-$(CH=CH)_2$ | 2 | $CH_3$ | |
| 1.104 | $CF_3$ | Br | 3,4-$(CH=CH)_2$ | 2 | $CH_2OC_2H_5$ | 110-112° |
| 1.105 | $C_2F_5$ | Br | 3,4-$(CH=CH)_2$ | 2 | $CH_2OC_2H_5$ | 131,5-133,5° |
| 1.106 | $C_2F_5$ | Br | 3,4-$(CH=CH)_2$ | 2 | $CH_3$ | |
| 1.107 | $CF_3$ | Br | 2,3-$(CH=CH)_2$ | 2 | $CH_3$ | |
| 1.108 | $CF_3$ | Br | 2,3-$(CH=CH)_2$ | 2 | $CH_2OC_2H_5$ | |
| 1.109 | $C_2F_5$ | Br | 2,3-$(CH=CH)_2$ | 2 | $CH_2OC_2H_5$ | |
| 1.110 | $C_2F_5$ | Br | 2,3-$(CH=CH)_2$ | 2 | $CH_3$ | |
| 1.111 | $CF_3$ | Br | 2,3-$(CH=CH)_2$,4-Cl | 3 | $CH_3$ | |
| 1.112 | $CF_3$ | Br | 2,3-$(CH=CH)_2$,4-Cl | 3 | $CH_2OC_2H_5$ | |
| 1.113 | $C_2F_5$ | Br | 2,3-$(CH=CH)_2$,4-Cl | 3 | $CH_3$ | |
| 1.114 | $C_2F_5$ | Br | 2,3-$(CH=CH)_2$,4-Cl | 3 | $CH_2OC_2H_5$ | |
| 1.115 | $CF_3$ | Br | 3,4-$OCF_2O$ | 2 | $CH_3$ | |
| 1.116 | $CF_3$ | Br | 3,4-$OCF_2O$ | 2 | $CH_2OC_2H_5$ | |
| 1.117 | $C_2F_5$ | Br | 3,4-$OCF_2O$ | 2 | $CH_2OC_2H_5$ | |
| 1.118 | $C_2F_5$ | Br | 3,4-$OCF_2O$ | 2 | $CH_3$ | |
| 1.119 | $CF_3$ | Br | 4-$CH_3SO_2$ | 1 | $CH_3$ | |
| 1.120 | $CF_3$ | Br | 4-$CH_3SO_2$ | 1 | $CH_2OC_2H_5$ | |
| 1.121 | $C_2F_5$ | Br | 4-$CH_3SO_2$ | 1 | $CH_2OC_2H_5$ | |

| Verb. Nr. | $R_1$ | $R_2$ | $R_3$ | n | $R_4$ | phys. Daten |
|---|---|---|---|---|---|---|
| 1.122 | $C_2F_5$ | Br | 4-$CH_3SO_2$ | 1 | $CH_3$ | |
| 1.123 | $CF_3$ | Br | 4-$CH_3S$ | 1 | $CH_3$ | |
| 1.124 | $CF_3$ | Br | 4-$CH_3S$ | 1 | $CH_2OC_2H_5$ | |
| 1.125 | $C_2F_5$ | Br | 4-$CH_3S$ | 1 | $CH_2OC_2H_5$ | |
| 1.126 | $C_2F_5$ | Br | 4-$CH_3S$ | 1 | $CH_3$ | |
| 1.127 | $CF_3$ | Br | 4-$CH_3$ | 1 | $CH_3$ | |
| 1.128 | $CF_3$ | Br | 4-$CH_3$ | 1 | $CH_2OC_2H_5$ | |
| 1.129 | $C_2F_5$ | Br | 4-$CH_3$ | 1 | $CH_2OC_2H_5$ | |
| 1.130 | $C_2F_5$ | Br | 4-$CH_3$ | 1 | $CH_3$ | |
| 1.131 | $CF_3$ | Br | 4-$OCH_3$ | 1 | $CH_3$ | |
| 1.132 | $CF_3$ | Br | 4-$OCH_3$ | 1 | $CH_2OC_2H_5$ | |
| 1.133 | $C_2F_5$ | Br | 4-$OC_2H_5$ | 1 | $CH_2OC_2H_5$ | |
| 1.134 | $C_2F_5$ | Br | 4-$OC_2H_5$ | 1 | $CH_3$ | |
| 1.135 | $CF_3$ | Br | 3-Cl | 1 | $CH_3$ | |
| 1.136 | $CF_3$ | Br | 3-Cl | 1 | $CH_2OC_2H_5$ | |
| 1.137 | $C_2F_5$ | Br | 3-Cl | 1 | $CH_2OC_2H_5$ | |
| 1.138 | $C_2F_5$ | Br | 3-Cl | 1 | $CH_3$ | |
| 1.139 | $CF_3$ | Br | 3-$CF_3$ | 1 | $CH_3$ | |
| 1.140 | $CF_3$ | Br | 3-$CF_3$ | 1 | $CH_2OC_2H_5$ | |
| 1.141 | $C_2F_5$ | Br | 3-$CF_3$ | 1 | $CH_2OC_2H_5$ | |
| 1.142 | $C_2F_5$ | Br | 3-$CF_3$ | 1 | $CH_3$ | |
| 1.143 | $CF_3$ | Br | 4-CN | 1 | $CH_3$ | |
| 1.144 | $CF_3$ | Br | 4-CN | 1 | $CH_2OC_2H_5$ | |
| 1.145 | $C_2F_5$ | Br | 4-CN | 1 | $CH_2OC_2H_5$ | |
| 1.146 | $C_2F_5$ | Br | 4-CN | 1 | $CH_3$ | |
| 1.147 | $CF_3$ | Br | 3,4$(Cl)_2$ | 2 | H | 207,5-209° |
| 1.148 | $CF_3$ | Cl | 4-Cl | 1 | H | 211,5-213° |
| 1.149 | $C_2F_5$ | Br | 3,4$(Cl)_2$ | 2 | H | 195,5-197° |
| 1.150 | $C_2F_5$ | Cl | 4-Cl | 1 | H | 195,5-197° |
| 1.151 | $CF_3$ | Br | 3,4-$(CH=CH)_2$ | 2 | H | 247-248° |
| 1.152 | $C_2F_5$ | Br | 3,4-$(CH=CH)_2$ | 2 | H | 197-198,5° |
| 1.153 | $CF_3$ | Br | 4-$OCF_3$ | 1 | H | 212,5-215° |

| Verb. Nr. | $R_1$ | $R_2$ | $R_3$ | n | $R_4$ | phys. Daten |
|---|---|---|---|---|---|---|
| 1.154 | $C_2F_5$ | Br | 4-$OCF_3$ | 1 | H | 209,5-211° |
| 1.155 | $CF_3$ | Cl | 4-$OCF_3$ | 1 | H | 214-214,5° |
| 1.156 | $C_2F_5$ | Cl | 4-$OCF_3$ | 1 | H | 206-208° |
| 1.157 | $CF_3$ | Cl | 4-$OCF_3$ | 1 | $CH_2OC_2H_5$ | $n_D^{23}$= 1,4761 |
| 1.158 | $C_2F_5$ | Cl | 4-$OCF_3$ | 1 | $CH_2OC_2H_5$ | $n_D^{22,5}$= 1,4636 |
| 1.159 | $CF_3$ | Cl | 4-F | 1 | H | |
| 1.160 | $CF_3$ | Cl | 4-F | 1 | $CH_2OC_2H_5$ | |
| 1.161 | $CF_3$ | Cl | 4-$CF_3$ | 1 | H | |
| 1.162 | $C_2F_5$ | Cl | 4-F | 1 | H | |
| 1.163 | $C_2F_5$ | Cl | 4-F | 1 | $CH_2OC_2H_5$ | |
| 1.164 | $CF_3$ | Cl | 2,4$(Cl)_2$ | 2 | H | |
| 1.165 | $C_2F_5$ | Cl | 2,4$(Cl)_2$ | 2 | H | |
| 1.166 | $CF_3$ | Cl | 3,4$(Cl)_2$ | 2 | H | |
| 1.167 | $C_2F_5$ | Cl | 3,4$(Cl)_2$ | 2 | H | |
| 1.168 | $CF_3$ | Cl | 4-Br | 1 | H | |
| 1.169 | $C_2F_5$ | Cl | 4-Br | 1 | H | |
| 1.170 | $CF_3$ | Cl | 4-Br | 1 | $CH_2OC_2H_5$ | |
| 1.171 | $C_2F_5$ | Cl | 4-Br | 1 | $CH_2OC_2H_5$ | |
| 1.172 | $CF_3$ | Br | 4-Br | 1 | H | |
| 1.173 | $C_2F_5$ | Br | 4-Br | 1 | H | |
| 1.174 | $CF_3$ | Br | 4-$OCF_2CHF_2$ | 1 | H | |
| 1.175 | $C_2F_5$ | Br | 4-$OCF_2CHF_2$ | 1 | H | |
| 1.176 | $CF_3$ | Cl | 4-$OCF_2CHF_2$ | 1 | H | |
| 1.177 | $CF_3$ | Cl | 4-$OCF_2CHF_2$ | 1 | $CH_2OC_2H_5$ | |
| 1.178 | $C_2F_5$ | Cl | 4-$OCF_2CHF_2$ | 1 | $CH_2OC_2H_5$ | |
| 1.179 | $CF_3$ | Br | 3,4-$OCF_2O$ | 2 | H | |
| 1.180 | $C_2F_5$ | Br | 3,4-$OCF_2O$ | 2 | H | |
| 1.181 | $CF_3$ | Cl | 3,4-$OCF_2O$ | 2 | H | |
| 1.182 | $CF_3$ | Cl | 3,4-$OCF_2O$ | 2 | $CH_2OC_2H_5$ | |
| 1.183 | $C_2F_5$ | Cl | 3,4-$OCF_2O$ | 2 | H | |
| 1.184 | $C_2F_5$ | Cl | 3,4-$OCF_2O$ | 2 | $CH_2OC_2H_5$ | |

| Verb. Nr. | $R_1$ | $R_2$ | $R_3$ | n | $R_4$ | phys. Daten |
|---|---|---|---|---|---|---|
| 1.185 | $CF_3$ | Br | 2,3-$OCF_2O$ | 2 | H | |
| 1.186 | $CF_3$ | Br | 2,3-$OCF_2O$ | 2 | $CH_2OC_2H_5$ | |
| 1.187 | $C_2F_5$ | Br | 2,3-$OCF_2O$ | 2 | H | |
| 1.188 | $C_2F_5$ | Br | 2,3-$OCF_2O$ | 2 | $CH_2OC_2H_5$ | |
| 1.189 | $CF_3$ | Cl | 2,3-$OCF_2O$ | 2 | H | |
| 1.190 | $CF_3$ | Cl | 2,3-$OCF_2O$ | 2 | $CH_2OC_2H_5$ | |
| 1.191 | $C_2F_5$ | Cl | 2,3-$OCF_2O$ | 2 | H | |
| 1.192 | $C_2F_5$ | Cl | 2,3-$OCF_2O$ | 2 | $CH_2OC_2H_5$ | |
| 1.193 | $C_2F_5$ | Br | 4-$OCH_3$ | 1 | $CH_3$ | |
| 1.194 | $C_2F_5$ | Br | 4-$OCH_3$ | 1 | $CH_2OC_2H_5$ | |

Tabelle 2

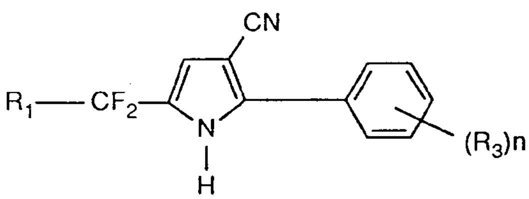

| Verb. Nr. | $R_1$ | $R_3$ | n | phys. Daten |
|---|---|---|---|---|
| 2.1 | $CF_3$ | 4-Cl | 1 | 223,5-224° |
| 2.2 | $C_2F_5$ | 4-Cl | 1 | 189,5-192° |
| 2.3 | $CF_3$ | 4-F | 1 | 223-226° |
| 2.4 | $C_2F_5$ | 4-F | 1 | 190,5-191° |
| 2.5 | $CF_3$ | 4-$CF_3$ | 1 | 230-231,5° |
| 2.6 | $C_2F_5$ | 4-$CF_3$ | 1 | 187,5-188,5° |
| 2.7 | $C_6F_{13}$-n | 4-Cl | 1 | 193-193,5° |
| 2.8 | $CF_3$ | 2,4$(Cl)_2$ | 2 | 164-166° |
| 2.9 | $C_2F_5$ | 2,4$(Cl)_2$ | 2 | 153-156° |
| 2.10 | $CF_3$ | 3,4$(Cl)_2$ | 2 | 196,5-198° |
| 2.11 | $C_2F_5$ | 3,4$(Cl)_2$ | 2 | 198-198,5° |
| 2.12 | $CF_3$ | 2,4,6$(Cl)_3$ | 3 | |
| 2.13 | $C_2F_5$ | 2,4,6$(Cl)_3$ | 3 | |
| 2.14 | $CF_3$ | 4-Br | 1 | |

| Verb. Nr. | $R_1$ | $R_3$ | n | phys. Daten |
|---|---|---|---|---|
| 2.15 | $C_2F_5$ | 4-Br | 1 | |
| 2.16 | $CF_3$ | 4-$OCF_3$ | 1 | 224,5-226° |
| 2.17 | $C_2F_5$ | 4-$OCF_3$ | 1 | 195,5-197° |
| 2.18 | $CF_3$ | 4-$OCF_2CHF_2$ | 1 | |
| 2.19 | $C_2F_5$ | 4-$OCF_2CHF_2$ | 1 | |
| 2.20 | $CF_3$ | 4-$OCF_2CHFCF_3$ | 1 | |
| 2.21 | $C_2F_5$ | 4-$OCF_2CHFCF_3$ | 1 | |
| 2.22 | $CF_3$ | 2,3,4,5,6$(F)_5$ | 5 | |
| 2.23 | $C_2F_5$ | 2,3,4,5,6$(F)_5$ | 5 | |
| 2.24 | $CF_3$ | 4-$OCH_2CF_3$ | 1 | |
| 2.25 | $C_2F_5$ | 4-$OCH_2CF_3$ | 1 | |
| 2.26 | $CF_3$ | 2,6$(Cl)_2$,4$CF_3$ | 3 | |
| 2.27 | $C_2F_5$ | 2,6$(Cl)_2$,4$CF_3$ | 3 | |
| 2.28 | $CF_3$ | 3,4-$(CH=CH)_2$ | 2 | 141,5-143° |
| 2.29 | $C_2F_5$ | 3,4-$(CH=CH)_2$ | 2 | 148-149° |
| 2.30 | $CF_3$ | 2,3-$(CH=CH)_2$ | 2 | |
| 2.31 | $C_2F_5$ | 2,3-$(CH=CH)_2$ | 2 | |
| 2.32 | $CF_3$ | 2,3-$(CH=CH)_2$,4Cl | 3 | |
| 2.33 | $C_2F_5$ | 2,3-$(CH=CH)_2$,4Cl | 3 | |
| 2.34 | $CF_3$ | 3,4-$OCF_2O$ | 2 | |
| 2.35 | $C_2F_5$ | 3,4-$OCF_2O$ | 2 | |
| 2.36 | $CF_3$ | 4-$CH_3SO_2$ | 1 | |
| 2.37 | $C_2F_5$ | 4-$CH_3SO_2$ | 1 | |
| 2.38 | $CF_3$ | 4-$CH_3S$ | 1 | |
| 2.39 | $C_2F_5$ | 4-$CH_3S$ | 1 | |
| 2.40 | $CF_3$ | 4-$CH_3$ | 1 | |
| 2.41 | $C_2F_5$ | 4-$CH_3$ | 1 | |
| 2.42 | $CF_3$ | 4-$OCH_3$ | 1 | |
| 2.43 | $C_2F_5$ | 4-$OCH_3$ | 1 | |
| 2.44 | $CF_3$ | 3-Cl | 1 | |
| 2.45 | $C_2F_5$ | 3-Cl | 1 | |
| 2.46 | $CF_3$ | 3-$CF_3$ | 1 | |
| 2.47 | $C_2F_5$ | 3-$CF_3$ | 1 | |

| Verb. Nr. | $R_1$ | $R_3$ | n | phys. Daten |
|---|---|---|---|---|
| 2.48 | $CF_3$ | 4-CN | 1 | |
| 2.49 | $C_2F_5$ | 4-CN | 1 | |
| 2.50 | $C_2F_5$ | $4\text{-}OC_2H_5$ | 1 | |
| 2.51 | $CF_3$ | $2,3\text{-}OCF_2O$ | 2 | |
| 2.52 | $C_2F_5$ | $2,3\text{-}OCF_2O$ | 2 | |

Formulierungsbeispiele (% = Gewichtsprozent)

| Beispiel F1: Emulsions-Konzentrate | a) | b) | c) |
|---|---|---|---|
| Wirkstoff Nr. 1.23 | 25 % | 40 % | 50 % |
| Ca-Dodecylbenzolsulfonat | 5 % | 8 % | 6 % |
| Ricinusölpolyethylenglykolether (36 Mol EO) | 5 % | - | - |
| Tributylphenolpolyethylenglykolether (30 Mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.23 | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.22 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Dichlormethan gelöst, auf den Träger aufgesprüht und das Lösungsmittel anschliessend im Vakuum abgedampft.

| Beispiel F4: Stäubemittel | a) | b) |
| --- | --- | --- |
| Wirkstoff Nr. 1.22 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
| --- | --- | --- | --- |
| Wirkstoff Nr. 1.2 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyethylenglykol-ether (7-8 Mol EO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
| --- | --- |
| Wirkstoff Nr. 1.3 | 10 % |
| Octylphenolpolyethylenglykolether (4-5 Mol EO) | 3 % |
| Ca-Dodecylbenzolsulfonat | 3 % |
| Ricinusölpolyglykolether (36 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
| --- | --- | --- |
| Wirkstoff Nr. 1.2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff Nr. 1.4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff Nr. 1.2 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff Nr. 1.3 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele (% = Gewichtsprozent, sofern nichts anderes angegeben)

A. Insektizide Wirkung

Beispiel B1: Wirkung gegen Nilaparvata lugens

Reispflanzen werden mit einer wässrigen Emulsionsspritzbrühe, die 400 ppm Wirkstoff enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten und auf unbehandelten Pflanzen wird die prozentuale Reduktion der Population (% Wirkung) bestimmt.

Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B2: Wirkung gegen Heliothis virescens (ovi-/larvizid)

Auf Baumwolle abgelegte Eier von Heliothis virescens werden mit einer wässrigen Emulsionsspritzbrühe, die 400 ppm Wirkstoff enthält, besprüht. Nach 8 Tagen werden der prozentuale Schlupf der Eier und die Ueberlebensraten der Raupen im Vergleich zu unbehandelten Kontrollansätzen ausgewertet (% Reduktion der Population).

Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B3: Wirkung gegen Heliothis virescens

Junge Sojapflanzen werden mit einer wässrigen Emulsionsspritzbrühe, die 400 ppm Wirkstoff enthält, besprüht. Nach dem Antrocknen des Spritzbelages werden die Pflanzen mit 10 Raupen des ersten Stadiums von Heliothis virescens besiedelt und in einen Plastikbehälter gegeben. 6 Tage später erfolgt die Auswertung. Aus den Vergleichen der Anzahl toter Raupen und des Frassschadens zwischen den behandelten und unbehandelten Pflanzen werden die prozentuale Reduktion der Population und des Frasssschadens (% Wirkung) bestimmt.
Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B4: Wirkung gegen Spodoptera littoralis

Junge Sojapflanzen werden mit einer wässrigen Emulsionsspritzbrühe, die 400 ppm Wirkstoff enthält, besprüht. Nach dem Antrocknen des Spritzbelags werden die Pflanzen mit 10 Raupen des dritten Stadiums von Spodoptera littoralis besiedelt und in einen Plastikbehälter gegeben. 3 Tage später erfolgt die Auswertung. Aus den Vergleichen der Anzahl toter Raupen und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B5: Wirkung gegen Aphis craccivora

Erbsenkeimlinge werden mit Aphis craccivora infiziert, anschliessend mit einer Spritzbrühe, die 400 ppm Wirkstoff enthält, besprüht und dann bei 20°C inkubiert. 3 und 6 Tage später erfolgt die Auswertung. Aus dem Vergleich der Anzahl toter Blattläuse auf den behandelten und auf unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B6: Wirkung gegen Crocidolomia binotalis

Junge Kohlpflanzen werden mit einer wässrigen Emulsionsspritzbrühe, die 400 ppm Wirkstoff enthält, besprüht. Nach dem Antrocknen des Spritzbelages werden die Kohlpflanzen mit 10 Raupen des dritten Stadiums von Crocidolmia binotalis besiedelt und in einen Plastikbehälter gegeben. 3 Tage später erfolgt die Auswertung. Aus den Vergleichen der Anzahl toter Raupen und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B7: Wirkung gegen Nilaparvata lugens (systemisch)

Töpfe mit Reispflanzen werden in eine wässrige Emulsionslösung, die 10 ppm Wirkstoff enthält, gestellt. Anschliessend werden die Pflanzen mit Larven des 2. und 3. Stadiums besiedelt. 6 Tage später erfolgt die Auswertung. Aus dem Vergleich der Anzahl Zikaden auf den behandelten und auf unbehandelten Pflanzen wird die prozentuale Reduktion der Population (% Wirkung) bestimmt.
Verbindungen der Tabelle 1 zeigen gute Wirkung in diesem Test. Insbesondere zeigt die Verbindung Nr. 1.4 eine Wirkung von mehr als 80%.

Beispiel B8: Wirkung gegen Diabrotica balteata

Maiskeimlinge werden mit einer wässrigen Emulsionsspritzbrühe, die 400 ppm Wirkstoff enthält, besprüht. Nach dem Antrocknen des Spritzbelags werden die Keimlinge mit 10 Larven des zweiten Stadiums von

Diabrotica balteata besiedelt und in einen Plastikbehälter gegeben. 6 Tage später erfolgt die Auswertung. Aus dem Vergleich der Anzahl toter Larven zwischen den behandelten und unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.2, 1.3, 1.4 und 1.14 eine Wirkung von mehr als 80%.

Beispiel B9: Wirkung gegen Anthonomus grandis

Junge Baumwollpflanzen werden mit einer wässrigen Emulsionsspritzbrühe, die 400 ppm Wirkstoff enthält, besprüht. Nach dem Antrocknen des Spritzbelags werden die Baumwollpflanzen mit 10 Adulten von Anthonomus grandis besiedelt und in einen Plastikbehälter gegeben. 3 Tage später erfolgt die Auswertung. Aus den Vergleichen der Anzahl toter Käfer und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B10: Wirkung gegen Plutella xylostella

Junge Kohlpflanzen werden mit einer wässrigen Emulsionsspritzbrühe, die 400 ppm Wirkstoff enthält, besprüht. Nach dem Antrocknen des Spritzbelags werden die Pflanzen mit 10 Raupen des dritten Stadiums von Plutella xylostella besiedelt und in einen Plastikbehälter gegeben. 3 Tage später erfolgt die Auswertung. Aus den Vergleichen der Anzahl toter Raupen und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabelle 1 zeigen gute Wirkung in diesem Test.

Beispiel B11: Wirkung gegen Aonidiella aurantii

Kartoffelknollen werden mit Wanderlarven ("Crawlern") von Aonidiella aurantii besiedelt. Nach etwa 2 Wochen werden die Kartoffeln in eine wässrige Emulsions- bzw. Suspensions-Spritzbrühe getaucht, die 400 ppm Wirkstoff enthält. Nach dem Abtrocknen der Knollen werden diese in einem Plastikbehälter inkubiert. Zur Auswertung wird 10 bis 12 Wochen später die Ueberlebensrate der Wanderlarven der ersten Folgegeneration der behandelten Population mit derjenigen von unbehandelten Kontrollansätzen verglichen. Verbindungen der Tabelle 1 zeigen in diesem Test gute Wirkung.

Beispiel B12: Wirkung gegen Bemisia tabaci

Buschbohnenpflanzen werden in Gazekäfige gestellt und mit Adulten von Bemisia tabaci besiedelt. Nach erfolgter Eiablage werden alle Adulten entfernt. 10 Tage später werden die Pflanzen mit den darauf befindlichen Nymphen mit einer wässrigen Emulsions-spritzbrühe, die 400 ppm Wirkstoff enthält, besprüht. Nach weiteren 14 Tagen wird der prozentuale Schlupf der Eier im Vergleich zu unbehandelten Kontrollansätzen ausgewertet.
Verbindungen der Tabelle 1 zeigen gute Wirkung in diesem Test.

Beispiel B13: Wirkung gegen Blattella germanica

In eine Petrischale wird so viel acetonische Lösung (0,1%) des Wirkstoffs gegeben, dass ihre Menge einer Aufwandmenge von 2 g/m$^2$ entspricht. Wenn das Lösungsmittel verdunstet ist, werden 20 Nymphen von Blattella germanica (letztes Nymphenstadium) in die Schale gegeben und 2 Stunden lang der Wirkung der Testsubstanz ausgesetzt. Dann werden die Nymphen mit $CO_2$ narkotisiert, in eine frische Petrischale gebracht und bei 25° und 50 bis 70% Luftfeuchtigkeit im Dunkeln gehalten. Nach 48 Stunden wird die insektizide Wirkung durch Ermittlung der Abtötungsrate bestimmt.
Verbindungen der Tabelle 1 zeigen gute Wirkung in diesem Test. Insbesondere zeigt die Verbindung Nr. 1.3 eine Wirkung von mehr als 80%.

Beispiel B14: Wirkung gegen Lucilia cuprina

Jeweils 30 bis 50 frisch abgelegte Eier von Lucilia cuprina werden in Reagensgläser gegeben, in denen zuvor 4 ml Nährmedium mit 1 ml Testlösung, die 16 ppm Wirkstoff enthält, vermischt worden sind. Nach Beimpfung des Kulturmediums werden die Testgläser mit einem Wattestopfen verschlossen und im Brutschrank 4 Tage bei 30° bebrütet. Im unbehandelten Medium entwickeln sich bis zu diesem Zeitpunkt ca. 1 cm lange Larven (Stadium 3). Ist die Testsubstanz aktiv, so sind die Larven zu diesem Zeitpunkt entweder tot oder deutlich zurückgeblieben. Die Auswertung erfolgt nach 96 Stunden.
Verbindungen der Tabelle 1 zeigen gute Wirkung in diesem Test.

Beispiel B15: Wirkung gegen Musca domestica

Ein Zuckerwürfel wird mit so viel Testsubstanz-Lösung behandelt, dass nach Trocknen über Nacht die Konzentration der Testsubstanz im Zucker 250 ppm beträgt. Der so behandelte Würfel wird mit einem nassen Wattebausch und 10 Adulten eines OP-resistenten Stammes von Musca domestica auf eine Aluminiumschale gelegt. Diese wird mit einem Becherglas abgedeckt und bei 25° inkubiert. Nach 24 Stunden wird die Mortalitätsrate bestimmt.
Verbindungen der Tabelle 1 zeigen gute Wirkung in diesem Test. Insbesondere zeigen die Verbindungen Nr. 1.3 und 1.4 eine Wirkung von mehr als 80%.

Beispiel B16: Wirkung gegen Ctenocephalides felis

20 bis 25 Floheier werden in eine waagerecht stehende 50 ml-Zellkulturflasche gegeben, in der zuvor 15 g Flohlarven-Nährmedium, welches 10 ppm Wirkstoff enthält, vorgelegt wurden. Die Flaschen werden in einem Brutschank bei 26-27° und 60-70 % Luftfeuchtigkeit bebrütet. Nach 21 Tagen wird die Anwesenheit von adulten Flöhen, nicht geschlüpften Puppen und Larven überprüft.
Verbindungen der Tabelle 1 zeigen gute Wirkung in diesem Test.

B. Akarizide Wirkung

Beispiel B17: Wirkung gegen Dermanyssus gallinae

In einen nach oben offenen Glasbehälter werden 2 bis 3 ml einer 100 ppm Wirkstoff enthaltenden Lösung und ca. 200 Individuen unterschiedlicher Entwicklungsstadien von Dermanyssus gallinae gegeben. Der Behälter wird mit einem Wattebausch verschlossen, 10 Minuten (bis zur vollständigen Benetzung der Milben) geschüttelt und dann kurzfristig umgekehrt, damit die restliche Testlösung von der Watte aufgenommen werden kann. Nach 3 Tagen wird die Mortalität der Milben (in Prozent) durch Auszählen der toten Individuen ermittelt.
Verbindungen der Tabelle 1 zeigen gute Wirkung in diesem Test.

Beispiel B18: Wirkung gegen Tetranychus urticae

Junge Bohnenpflanzen werden mit einer Mischpopulation von Tetranychus urticae besiedelt und 1 Tag später mit einer wässrigen Emulsionsspritzbrühe, die 400 ppm Wirkstoff enthält, besprüht Die Pflanzen werden anschliessend 6 Tage bei 25°C inkubiert und danach ausgewertet. Aus den Vergleichen der Anzahl toter Eier, Larven und Adulten auf den behandelten und auf unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.2, 1.3 und 1.4 eine Wirkung von mehr als 80%.

Beispiel B19: Wirkung gegen Boophilus microplus

Vollgesogene adulte Zeckenweibchen werden auf eine PVC-Platte aufgeklebt und mit einem Wattebausch überdeckt. Zur Behandlung werden die Testtiere mit 10 ml einer wässrigen Testlösung, die 125 ppm Wirkstoff enthält, übergossen. Anschliessend wird der Wattebausch entfernt und die Zecken werden 4 Wochen zur Eiablage inkubiert. Die Wirkung gegen Boophilus microplus zeigt sich entweder beim Weibchen als Mortalität oder Sterilität oder bei den Eiern als ovizide Wirkung.
Verbindungen der Tabelle 1 zeigen gute Wirkung in diesem Test.

C. Fungizide Wirkung

Beispiel B20: Wirkung gegen Puccinia graminis auf Weizen

a) Residual-protektive Wirkung

Testmethode: Weizenpflanzen werden 6 Tage nach Aussaat mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. Die Beurteilung der Rostpustelnentwicklung 12 Tage nach Infektion führt zur Bewertung der Wirkung der Aktivsubstanz. Testresultat: Verbindungen der Tabelle 1 zeigen gegen Puccinia graminis auf Weizen gute residual-protektive Wirkung, z. B. reduzieren die Verbindungen Nr. 1.2 und 1.3 den Pilzbefall auf 20 bis 5%. Nicht mit der Aktivsubstanz behandelte infizierte Kontrollpflanzen weisen dagegen einen Pilzbefall von 100% auf.

b) Systemische Wirkung

Testmethode: Zu Weizenpflanzen wird 5 Tage nach Aussaat eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. Die Beurteilung der Rostpustelnentwicklung 12 Tage nach Infektion führt zur Bewertung der Wirkung der Aktivsubstanz. Testresultat: Verbindungen der Tabelle 1 zeigen gegen Puccinia graminis auf Weizen gute systemische Wirkung.

Beispiel B21: Wirkung gegen Phytophthora infestans auf Tomaten

a) Residual-protektive Wirkung

Testmethode: Tomatenpflanzen werden nach dreiwöchiger Anzucht mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls 5 Tage nach Infektion, während denen 90 bis 100 Prozent relative Luftfeuchtigkeit und eine Temperatur von 20° aufrechterhalten werden, führt zur Bewertung der Wirkung der Aktivsubstanz.
Testresultat: Verbindungen der Tabelle 1 zeigen gegen Phytophthora infestans auf Tomaten gute residual-protektive Wirkung, z. B. reduzieren die Verbindungen Nr. 1.2, 1.3 und 1.4 den Pilzbefall auf 20 bis 0%. Nicht mit der Aktivsubstanz behandelte infizierte Kontrollpflanzen weisen dagegen einen Pilzbefall von 10% auf.

b) Systemische Wirkung

Testmethode: Zu Tomatenpflanzen wird nach dreiwöchiger Anzucht eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. Nach 48 Stunden werden die Pflanzen mit einer Sporangiensuspension des Pilzes infiziert. Die Beurteilung des Pilzbefalls 5 Tage nach Infektion, während denen 90 bis 100 Prozent relative Luftfeuchtigkeit und eine Temperatur von 20° aufrechterhalten werden, führt zur Bewertung der Wirkung der Aktivsubstanz.
Testresultat: Verbindungen der Tabelle 1 zeigen gegen Phytophthora infestans auf Tomaten gute systemische Wirkung.

Beispiel B22: Residual-protektive Wirkung gegen Cercospora arachidicola auf Erdnüssen

Testmethode: 10 bis 15 cm hohe Erdnusspflanzen werden mit einer aus einem einen der erfindungsgemässen Wirkstoffe enthaltenden Spritzpulver hergestellten wässrigen Spritzbrühe (0,02% Aktivsubstanz) tropfnass besprüht und 48 Stunden später mit einer Konidiensuspension des Pilzes infiziert. Die Pflanzen werden 72 Stunden bei 21° und hoher Luftfeuchtigkeit inkubiert und anschliessend bis zum Auftreten der typischen Blattflecken in einem Gewächshaus aufgestellt. Die Bewertung der Wirkung der Aktivsubstanz erfolgt 12 Tage nach Infektion aufgrund von Anzahl und Grösse der Blattflecken.
Testresultat: Verbindungen der Tabelle 1 zeigen gegen Cercospora arachidicola auf Erdnüssen gute residual-protektive Wirkung.

Beispiel B23: Wirkung gegen Plasmopara viticola auf Reben

a) Residual-protektive Wirkung

Testmethode: Rebensämlinge im 4 bis 5 Blatt-Stadium werden mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls 6 Tage nach Infektion, während denen 95 bis 100 Prozent relative Luftfeuchtigkeit und eine Temperatur von 20° aufrechterhalten werden, führt zur Bewertung der Wirkung der Aktivsubstanz.
Testresultat: Verbindungen der Tabelle 1 zeigen gegen Plasmopara viticola auf Reben gute präventive residual-protektive Wirkung, z. B. reduzieren die Verbindungen Nr. 1.2, 1.3 und 1.4 den Pilzbefall auf 20 bis 5%. Nicht mit der Aktivsubstanz behandelte infizierte Kontrollpflanzen weisen dagegen einen Pilzbefall von 100% auf.

b) Residual-protektive Wirkung

Testmethode: Rebensämlinge im 4 bis 5 Blatt-Stadium werden mit einer Sporangiensuspension des Pilzes infiziert, 24 Stunden in einer Feuchtkammer (Bedingungen: 95 bis 100 Prozent relative Luftfeuchtigkeit bei 20°) inkubiert und dann mit einer aus einem einen der erfindungsgemässen Wirkstoffe enthaltenden Spritzpulver hergestellten wässrigen Spritzbrühe (0,02% Aktivsubstanz) tropfnass besprüht. Nach Antrocknen des Spritzbelags werden die Pflanzen wieder in die Feuchtkammer gebracht. Die Beurteilung des Pilzbefalls 6 Tage nach Infektion führt zur Bewertung der Wirkung der Aktivsubstanz.
Testresultat: Verbindungen der Tabelle 1 zeigen gegen Plasmopara viticola auf Reben gute kurative residual-protektive Wirkung, z. B. reduzieren die Verbindungen Nr. 1.2, 1.3 und 1.4 den Pilzbefall auf 20 bis 5%. Nicht mit der Aktivsubstanz behandelte infizierte Kontrollpflanzen weisen dagegen einen Pilzbefall von 100% auf.

Beispiel B24: Wirkung gegen Erysiphe graminis auf Gerste

a) Residual-protektive Wirkung

Testmethode: Ungefähr 8 cm hohe Gerstenpflanzen werden mit einer aus einem einen der erfindungsgemässen Wirkstoffe enthaltenden Spritzpulver hergestellten wässrigen Spritzbrühe (0,02% Aktivsubstanz) tropfnass besprüht und 3 bis 4 Stunden später mit Konidien des Pilzes bestäubt. Die infizierten Pflanzen werden in einem Gewächshaus bei 22° aufgestellt. Die Beurteilung des Pilzbefalls 10 Tage nach Infektion führt zur Bewertung der Wirkung der Aktivsubstanz.
Testresultat: Verbindungen der Tabelle 1 zeigen gegen Erysiphe graminis auf Gerste gute residual-protektive Wirkung, z. B. reduzieren die Verbindungen Nr. 1.2, 1.3 und 1.4 den Pilzbefall auf 20 bis 5%. Nicht mit der Aktivsubstanz behandelte infizierte Kontrollpflanzen weisen dagegen einen Pilzbefall von 100% auf.

b) Systemische Wirkung

Testmethode: Zu ungefähr 8 cm hohen Gerstenpflanzen wird eine aus einem einen der erfindungsgemässen Wirkstoffe enthaltenden Spritzpulver hergestellte wässrige Spritzbrühe (0,002% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit Konidien des Pilzes bestäubt. Die infizierten Pflanzen werden in einem Gewächshaus bei 22° aufgestellt. Die Beurteilung des Pilzbefalls 10 Tage nach Infektion führt zur Bewertung der Wirkung der Aktivsubstanz.
Testresultat: Verbindungen der Tabelle 1 zeigen gegen Erysiphe graminis auf Gerste gute systemische Wirkung.

**Patentansprüche**

1. Eine Verbindung der Formel

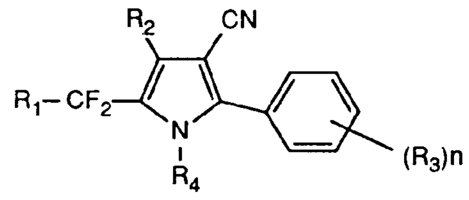

(I),

worin

n die Zahl 1, 2, 3, 4 oder 5, wobei, wenn n grösser als 1 ist, die Reste $R_3$ gleich oder verschieden sind;

$R_1$ Halogen-$C_1$-$C_9$-akyl;

$R_2$ Halogen;

$R_3$ Wasserstoff, Cyano, Nitro, Halogen, $C_1$-$C_4$-Alkyl, $C_1$-$C_4$-Alkoxy, $C_1$-$C_4$-Akylthio, $C_1$-$C_4$-Alkansulfinyl, $C_1$-$C_4$-Alkansulfonyl, Halogen-$C_1$-$C_4$-alkyl, Halogen-$C_1$-$C_4$-alkoxy, Halogen-$C_1$-$C_4$-akylthio, Halogen-$C_1$-$C_4$-alkansulfinyl oder Halogen-$C_1$-$C_4$-alkansulfonyl und/oder zwei an benachbarte C-Atome des Phenylrings gebundene Substituenten $R_3$ gemeinsam eine unsubstituierte oder durch Halogen, $C_1$-$C_4$-Alkyl, $C_1$-$C_4$-Alkoxy oder Halogen-$C_1$-$C_4$-alkyl substituierte Brücke, ausgewählt aus der Gruppe von Brücken, bestehend aus -O-$CH_2$-O-, -O-$CH_2$-$CH_2$-O-, -O-$CH_2$-$CH_2$-, -O-$CH_2$-$CH_2$-$CH_2$- und -CH=CH-CH=CH-; und

$R_4$ Wasserstoff, $C_1$-$C_4$-Akyl, $C_3$-$C_4$-Alkenyl, Halogen-$C_3$-$C_4$-alkenyl, $C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl, Di-$C_1$-$C_4$-alkylaminocarbonyl oder eine durch einen oder mehrere Substituenten, ausgewählt aus der Gruppe, bestehend aus Halogen, $C_1$-$C_4$-Alkoxy, Hydroxy, $C_1$-$C_4$-Alkylthio, $C_2$-$C_6$-Alkoxyalkoxy, $C_1$-$C_4$-Alkansulfinyl, $C_1$-$C_4$-Alkansulfonyl, $C_1$-$C_4$-Akylcarbonyl, $C_1$-$C_4$-Alkoxycarbonyl, $C_1$-$C_4$-Alkylcarbonyloxy, Cyano, $C_2$-$C_4$-Alkenylcarbonyloxy, Phenyl, Phenoxy, Phenylthio, Benzyloxy, Benzoyloxy, Benzoyl, Phenylsulfinyl und Phenylsulfonyl, wobei die in diesen Phenyl-, Phenoxy-, Phenylthio-, Benzyloxy-, Benzoyloxy-, Benzoyl-, Phenylsulfinyl- und Phenylsulfonyl-Substituenten enthaltenen Phenylkerne jeweils durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio, $C_1$-$C_4$-Alkansulfinyl, $C_1$-$C_4$-Alkansulfonyl, Halogen-$C_1$-$C_4$-alkylthio, Halogen-$C_1$-$C_4$-alkansulfinyl, Halogen-$C_1$-$C_4$-alkansulfonyl, Cyano oder Nitro substituiert sein können, substituierte $C_1$-$C_4$-Alkylgruppe bedeuten,

in freier Form oder in Salzform.

2. Eine Verbindung gemäss Anspruch 1 der Formel I in freier Form.

3. Eine Verbindung gemäss Anspruch 2 der Formel I, worin $R_1$ Perfluor-$C_1$-$C_6$-alkyl bedeutet.

4. Eine Verbindung gemäss Anspruch 2 der Formel I, worin $R_2$ Chlor oder Brom ist.

5. Eine Verbindung gemäss Anspruch 2 der Formel I, worin n die Zahl 1, 2, 3, 4 oder 5, wobei, wenn n grösser als 1 ist, die Reste $R_3$ gleich oder verschieden sind, und $R_3$ Halogen, Halogen-$C_1$-$C_4$-alkyl, Halogen-$C_1$-$C_4$-alkoxy, Cyano, $C_1$-$C_4$-Akyl, $C_1$-$C_4$-Alkoxy, $C_1$-$C_4$-Alkylthio oder $C_1$-$C_4$-Alkansulfonyl und/oder zwei an benachbarte C-Atome des Phenylrings gebundene Substituenten $R_3$ gemeinsam -O-C(Halogen)$_2$-O-, insbesondere -O-$CF_2$-O-, oder -CH=CH-CH=CH- bedeuten.

6. Eine Verbindung gemäss Anspruch 2 der Formel I, worin $R_4$ Wasserstoff, $C_1$-$C_4$-Akyl, $C_3$-$C_4$-Alkenyl, Halogen-$C_3$-$C_4$-alkenyl, $C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl, Di-$C_1$-$C_4$-akylaminocarbonyl oder eine durch einen oder mehrere Substituenten, ausgewählt aus der Gruppe, bestehend aus Halogen, $C_1$-$C_4$-Alkoxy, Hydroxy, $C_1$-$C_4$-Alkylthio, $C_2$-$C_6$-Alkoxyalkoxy, $C_1$-$C_4$-Alkansulfinyl, $C_1$-$C_4$-Alkansulfonyl, $C_1$-$C_4$-Alkylcarbonyl, $C_1$-$C_4$-Alkoxycarbonyl, $C_1$-$C_4$-Alkylcarbonyloxy, Cyano, $C_2$-$C_4$-Alkenylcarbonyloxy, Phenyl, Phenoxy, Phenylthio, Benzyloxy, Benzoyloxy, Benzoyl, Phenylsulfinyl und Phenylsulfonyl, wobei die in diesen Phenyl-, Phenoxy-, Phenylthio-, Benzyloxy-, Benzoyloxy-, Benzoyl-, Phenylsulfinyl- und Phenylsulfonyl-Substituenten enthaltenen Phenylkerne jeweils durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio, $C_1$-$C_4$-Alkansulfonyl, Halogen-$C_1$-$C_4$-alkylthio, Halogen-$C_1$-$C_4$-alkansulfinyl, Halogen-$C_1$ -$C_4$-alkansulfonyl, Cyano oder Nitro substituiert sein können, substituierte $C_1$-$C_4$-Alkylgruppe bedeutet.

7. Eine Verbindung gemäss Anspruch 2 der Formel I, worin $R_4$ Wasserstoff, $C_1$-$C_4$-Akyl, $C_3$-$C_4$-Alkinyl, $C_1$-$C_4$-Alkansulfonyl, Di-$C_1$-$C_4$-alkylaminosulfonyl oder eine durch $C_1$-$C_4$-Alkoxy, $C_1$-$C_4$-Alkylthio, $C_2$-$C_6$-Alkoxyalkoxy, $C_1$-$C_4$-Alkansulfonyl, $C_1$-$C_4$-Alkylcarbonyl, $C_1$-$C_4$-Alkoxycarbonyl, Cyano, Monohalogenphe-

nyl oder Phenoxy substituierte $C_1$-$C_4$-Akylgruppe bedeutet.

8. Eine Verbindung gemäss Anspruch 2 der Formel I, worin $R_1$ Perfluor-$C_1$-$C_6$-alkyl, $R_2$ Chlor oder Brom, $R_3$ Halogen, Halogen-$C_1$-$C_4$-alkyl, Halogen-$C_1$-$C_4$-alkoxy, Cyano, $C_1$-$C_4$-Alkyl, $C_1$-$C_4$-Alkoxy, $C_1$-$C_4$-Alkyltio oder $C_1$-$C_4$-Alkansulfonyl, $R_4$ Wasserstoff, $C_1$-$C_4$-Alkyl oder $C_2$-$C_6$-Alkoxyalkyl und n die Zahl eins, zwei oder drei bedeuten, wobei n die Zahl eins, zwei, drei, vier oder fünf bedeuten kann, wenn alle Reste $R_3$ gleichzeitig für Halogen stehen.

9. Eine Verbindung gemäss Anspruch 8 der Formel I, worin $R_1$ Trifluormethyl oder Pentafluorethyl, $R_2$ Brom, $R_3$ Fluor, Chlor, Trifluormethyl, Fluor-$C_1$-$C_3$-alkoxy, Methylthio, Cyano oder $C_1$-$C_3$-Alkoxy, $R_4$ Wasserstoff, Methyl, Methoxymethyl oder Ethoxymethyl und n die Zahl eins, zwei oder drei bedeuten.

10. Eine Verbindung gemäss Anspruch 8 der Formel I, worin $R_1$ Trifluormethyl, $R_2$ Chlor oder Brom, $R_3$ Fluor, Chlor, Trifluormethyl, Fluor-$C_1$-$C_3$-alkoxy, Methylthio, Cyano oder $C_1$-$C_3$-Alkoxy, $R_4$ Wasserstoff, Methyl, Methoxymethyl oder Ethoxymethyl und n die Zahl eins, zwei oder drei bedeuten.

11. Eine Verbindung gemäss Anspruch 9 der Formel I, worin n die Zahl 1, $R_1$ Trifluormethyl oder Pentafluorethyl, $R_2$ Brom, $R_3$ Trifluormethyl, Trifluormethoxy oder Chlor und $R_4$ Wasserstoff oder Ethoxymethyl bedeuten.

12. Eine Verbindung gemäss Anspruch 2 der Formel I, ausgewählt aus der Gruppe, bestehend aus den Verbindungen
(a) 4-Brom-2-(4-chlorphenyl)-3-cyano-5-heptafluorpropyl-pyrrol,
(b) 4-Brom-2-(4-chlorphenyl)-3-cyano-1-ethoxymethyl-5-pentafluorethyl-pyrrol,
(c) 4-Brom-2-(4-chlorphenyl)-3-cyano-1-ethoxymethyl-5-heptafluorpropyl-pyrrol,
(d) 4-Chlor-2-(4-chlorphenyl)-3-cyano-5-heptafluorpropyl-pyrrol,
(e) 4-Chlor-2-(4-chlorphenyl)-3-cyano-1-ethoxymethyl-5-pentafluorethyl-pyrrol,
(f) 4-Chlor-2-(4-chlorphenyl)-3-cyano-1-ethoxymethyl-5-heptafluorpropyl-pyrrol,
(g) 4-Brom-3-cyano-5-pentafluorethyl-2-(4-trifluormethylphenyl)-pyrrol,
(h) 4-Chlor-3-cyano-5-pentafluorethyl-2-(4-trifluormethylphenyl)-pyrrol,
(i) 4-Brom-3-cyano-1-ethoxymethyl-5-pentafluorethyl-2-(4-trifluormethylphenyl)-pyrrol,
(j) 4-Chlor-3-cyano-1-ethoxymethyl-5-pentafluorethyl-2-(4-trifluormethylphenyl)-pyrrol,
(k) 4-Brom-3-cyano-5-pentafluorethyl-2-(4-trifluormethoxyphenyl)-pyrrol,
(l) 4-Chlor-3-cyano-5-pentafluorethyl-2-(4-trifluormethoxyphenyl)-pyrrol,
(m) 4-Brom-3-cyano-1-ethoxymethyl-5-pentafluorethyl-2-(4-trifluormethoxyphenyl)-pyrrol und
(n) 4-Chlor-3-cyano-1-ethoxymethyl-5-pentafluorethyl-2-(4-trifluormethoxyphenyl)-pyrrol.

13. Verfahren zur Herstellung einer Verbindung gemäss Anspruch 1 der Formel I, in freier Form oder in Salzform, dadurch gekennzeichnet, dass man
a) in die 4-Position des Pyrrolrings einer Verbindung der Formel

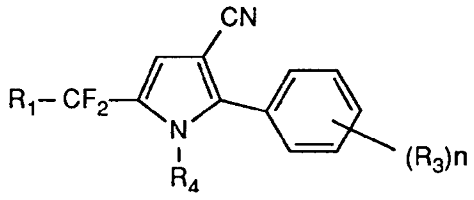

(II),

worin $R_1$ , $R_3$, $R_4$ und n die für die Formel I angegebenen Bedeutungen haben, oder ein Salz davon durch Umsetzung mit einem Halogenierungsmittel, vorzugsweise in Gegenwart einer Base, den Halogensubstituenten $R_2$ einführt oder
b) zur Herstellung einer Verbindung der Formel I, worin $R_4$ von Wasserstoff verschieden ist, oder eines Salzes davon eine, z. B. gemäss der Variante a) erhältliche, Verbindung der Formel I, worin $R_4$ Wasserstoff ist, oder ein Salz davon, vorzugsweise in Gegenwart einer Base, mit einer Verbindung der Formel

$$X\text{-}R_4 \qquad (III),$$

die bekannt ist oder in Analogie zu entsprechenden bekannten Verbindungen hergestellt werden kann und worin $R_4$ eine der für die Formel I angegebenen Bedeutungen mit Ausnahme von Wasserstoff hat und X für eine Abgangsgruppe steht, oder gegebenenfalls einem Salz davon umsetzt oder

c) zur Herstellung einer Verbindung der Formel I, worin $R_4$ Methyl oder eine Gruppe $R_2CH_2$- ist, oder eines Salzes davon eine Verbindung der Formel

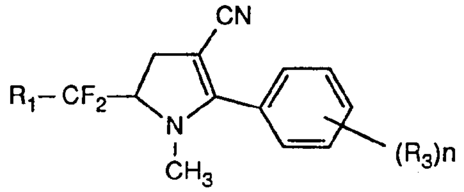

(VII),

worin $R_1$, $R_3$ und n die für die Formel I angegebenen Bedeutungen haben, oder ein Salz davon mit einem Halogenierungsmittel, gegebenenfalls in Gegenwart einer Base, gegebenenfalls in Gegenwart eines Radikalstarters, umsetzt und jeweils, wenn erwünscht, eine verfahrensgemäss oder auf andere Weise erhältliche Verbindung der Formel I, in freier Form oder in Salzform, in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

**14.** Schädlingsbekämpfungsmittel, dadurch gekennzeichnet, dass es mindestens eine Verbindung gemäss Anspruch 1 der Formel I, in freier Form oder in agrochemisch verwendbarer Salzform, als Wirkstoff und gegebenenfalls mindestens einen Hilfsstoff enthält.

**15.** Mittel gemäss Anspruch 14 zur Bekämpfung von Insekten, Vertretern der Ordnung Akarina und/oder phytopathogenen Pilzen.

**16.** Verfahren zur Herstellung eines mindestens einen Hilfsstoff enthaltenden Mittels gemäss Anspruch 14, dadurch gekennzeichnet, dass man den Wirkstoff mit dem (den) Hilfsstoff(en) innig vermischt und/oder vermahlt.

**17.** Verwendung einer Verbindung gemäss Anspruch 1 der Formel I, in freier Form oder in agrochemisch verwendbarer Salzform, zur Herstellung eines Mittels gemäss Anspruch 14.

**18.** Verwendung eines Mittels gemäss Anspruch 14 zur Bekämpfung von Schädlingen.

**19.** Verwendung gemäss Anspruch 18 zur Bekämpfung von Insekten, Vertretern der Ordnung Akarina und/oder phytopathogenen Pilzen.

**20.** Verwendung gemäss Anspruch 18 zum Schutz von pflanzlichem Vermehrungsgut.

**21.** Verfahren zur Bekämpfung von Schädlingen, dadurch gekennzeichnet, dass man ein Mittel gemäss Anspruch 14 auf die Schädlinge oder ihren Lebensraum appliziert.

**22.** Verfahren gemäss Anspruch 21 zur Bekämpfung von Insekten, Vertretern der Ordnung Akarina und/oder phytopathogenen Pilzen.

**23.** Verfahren gemäss Anspruch 21 zum Schutz von pflanzlichem Vermehrungsgut, dadurch gekennzeichnet, dass man das Vermehrungsgut oder den Ort der Ausbringung des Vermehrungsguts behandelt.

**24.** Pflanzliches Vermehrungsgut, behandelt gemäss dem in Anspruch 23 beschriebenen Verfahren.

**25.** Eine Verbindung der Formel

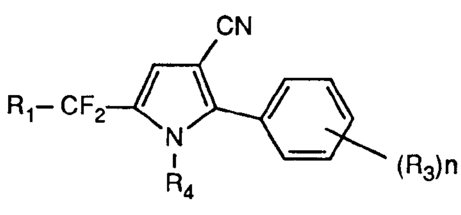

(II),

worin $R_1$, $R_3$, $R_4$ und n die für die Formel I angegebenen Bedeutungen haben, in freier Form oder in Salzform.

**26.** Verfahren zur Herstellung einer Verbindung gemäss Anspruch 25 der Formel II, in freier Form oder in Salzform, dadurch gekennzeichnet, dass man

d) zur Herstellung einer Verbindung der Formel II, worin $R_4$ Wasserstoff ist, oder eines Salzes davon eine Verbindung der Formel

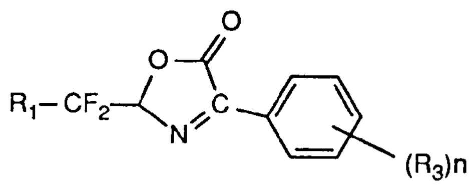

(IV),

worin $R_1$, $R_3$ und n die für die Formel I angegebenen Bedeutungen haben, oder ein Salz davon, vorzugsweise in einem hochsiedenden polaren Lösungsmittel, wie Nitromethan, und bei der Rückflusstemperatur des Reaktionsgemisches, mit 2-Chloracrylnitril umsetzt oder

e) zur Herstellung einer Verbindung der Formel II, worin $R_4$ von Wasserstoff verschieden ist, oder eines Salzes davon eine, z. B. gemäss der Variante d) erhältliche, Verbindung der Formel II, worin $R_4$ Wasserstoff ist, oder ein Salz davon, vorzugsweise in Gegenwart einer Base, z. B. von der unter der Variante a) angegebenen Art, mit einer Verbindung der Formel

$X\text{-}R_4$ (III),

die bekannt ist oder in Analogie zu entsprechenden bekannten Verbindungen hergestellt werden kann und worin $R_4$ eine der für die Formel I angegebenen Bedeutungen mit Ausnahme von Wasserstoff hat und X für eine Abgangsgruppe, z. B. von der unter der Variante b) angegebenen Art, steht, oder gegebenenfalls einem Salz davon umsetzt und jeweils, wenn erwünscht, eine verfahrensgemäss oder auf andere Weise erhältliche Verbindung der Formel II, in freier Form oder in Salzform, in eine andere Verbindung der Formel II überführt, ein verfahrensgemäss erhältliches Gemisch von Isomeren auftrennt und das gewünschte Isomere isoliert und/oder eine verfahrensgemäss erhältliche freie Verbindung der Formel II in ein Salz oder ein verfahrensgemäss erhältliches Salz einer Verbindung der Formel II in die freie Verbindung der Formel II oder in ein anderes Salz überführt.

**27.** Verwendung einer Verbindung gemäss Anspruch 25 der Formel II, in freier Form oder in Salzform, zur Herstellung einer Verbindung gemäss Anspruch 1.

**28.** Eine Verbindung der Formel

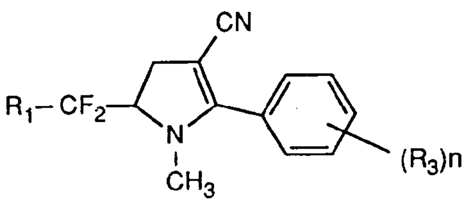

(VII),

worin $R_1$, $R_3$ und n die für die Formel I angegebenen Bedeutungen haben, in freier Form oder in Salzform.

**29.** Verfahren zur Herstellung einer Verbindung gemäss Anspruch 28 der Formel VII, in freier Form oder in Salzform, dadurch gekennzeichnet, dass man

f) eine Verbindung der Formel

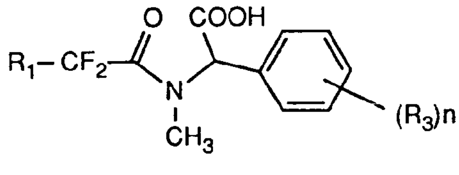

(VIII),

worin $R_1$, $R_3$ und n die für die Formel I angegebenen Bedeutungen haben, oder ein Salz davon, vorzugsweise in einem hochsiedenden polaren Lösungsmittel, wie Acetonitril, in Gegenwart eines Säureanhydrids, wie in Gegenwart von Acetanhydrid, und bei der Rückflusstemperatur des Reaktionsgemisches, mit Acrylnitril umsetzt und, wenn erwünscht, eine verfahrensgemäss oder auf andere Weise erhältliche Verbindung der Formel VII, in freier Form oder in Salzform, in eine andere Verbindung der Formel VII überführt, ein verfahrensgemäss erhältliches Gemisch von Isomeren auftrennt und das gewünschte Isomere isoliert und/oder eine verfahrensgemäss erhältliche freie Verbindung der Formel VII in ein Salz oder ein verfahrensgemäss erhältliches Salz einer Verbindung der Formel VII in die freie Verbindung der Formel VII oder in ein anderes Salz überführt.

**30.** Verwendung einer Verbindung gemäss Anspruch 28 der Formel VII, in freier Form oder in Salzform, zur Herstellung einer Verbindung gemäss Anspruch 1.

**Patentansprüche für folgenden Vertragsstaat : ES**

**1.** Verfahren zur Herstellung einer Verbindung der Formel

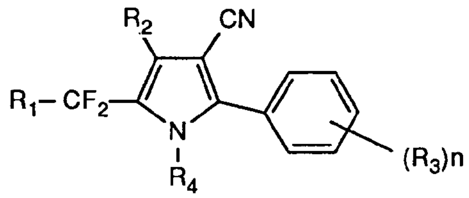

(I),

worin

n die Zahl 1, 2, 3, 4 oder 5, wobei, wenn n grösser als 1 ist, die Reste $R_3$ gleich oder verschieden sind;

$R_1$ Halogen-$C_1$-$C_9$-alkyl;

$R_2$ Halogen;

$R_3$ Wasserstoff, Cyano, Nitro, Halogen, $C_1$-$C_4$-Alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen-$C_1$-$C_4$-alkyl, Halogen-$C_1$-$C_4$-alkoxy, Halogen-$C_1$-$C_4$-alkylthio, Halogen-$C_1$-$C_4$-alkansulfinyl oder Halogen-$C_1$-$C_4$-alkansulfonyl und/oder zwei an benachbarte C-Atome des Phenylrings gebundene Substituenten $R_3$ gemeinsam eine unsubstituierte oder durch Halogen, $C_1$-$C_4$-Akyl, $C_1$-$C_4$-Alkoxy oder Halogen-$C_1$-$C_4$-alkyl substituierte Brücke, ausgewählt aus der Gruppe von Brücken, bestehend aus -O-$CH_2$-O-, -O-$CH_2$-$CH_2$-O-, -O-$CH_2$-$CH_2$-, -O-$CH_2$-$CH_2$-$CH_2$- und -CH=CH-CH=CH-; und

$R_4$ Wasserstoff, $C_1$-$C_4$-Akyl, $C_3$-$C_4$-Alkenyl, Halogen-$C_3$-$C_4$-alkenyl, $C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl, Di-$C_1$-$C_4$-alkylaminocarbonyl oder eine durch einen oder mehrere Substituenten, ausgewählt aus der Gruppe, bestehend aus Halogen, $C_1$-$C_4$-Alkoxy, Hydroxy, $C_1$-$C_4$-Alkylthio, $C_2$-$C_6$-Alkoxyalkoxy, $C_1$-$C_4$-Alkansulfinyl, $C_1$-$C_4$-Alkansulfonyl, $C_1$-$C_4$-Akylcarbonyl, $C_1$-$C_4$-Alkoxycarbonyl, $C_1$-$C_4$-Alkylcarbonyloxy, Cyano, $C_2$-$C_4$-Alkenylcarbonyloxy, Phenyl, Phenoxy, Phenylthio, Benzyloxy, Benzoyloxy, Benzoyl, Phenylsulfinyl und Phenylsulfonyl, wobei die in diesen Phenyl-, Phenoxy-, Phenylthio-, Benzyloxy-, Benzoyloxy-, Benzoyl-, Phenylsulfinyl- und Phenylsulfonyl-Substituenten enthaltenen Phenylkerne jeweils durch Halogen, $C_1$-$C_1$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio, $C_1$-$C_4$-Alkansulfinyl, $C_1$-$C_4$-Alkansulfonyl, Halogen-$C_1$-$C_4$-alkylthio, Halogen-$C_1$-$C_4$-alkansulfinyl, Halogen-$C_1$-$C_4$-alkansulfonyl, Cyano oder Nitro substituiert sein können, substituierte $C_1$-$C_4$-Akylgruppe bedeuten,

in freier Form oder in Salzform, dadurch gekennzeichnet, dass man

a) in die 4-Position des Pyrrolrings einer Verbindung der Formel

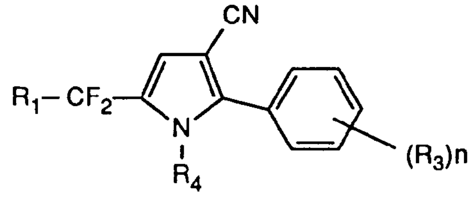

(II),

worin $R_1$, $R_3$, $R_4$ und n die für die Formel I angegebenen Bedeutungen haben, oder ein Salz davon durch

Umsetzung mit einem Halogenierungsmittel, vorzugsweise in Gegenwart einer Base, den Halogensubstituenten $R_2$ einführt oder
b) zur Herstellung einer Verbindung der Formel I, worin $R_4$ von Wasserstoff verschieden ist, oder eines Salzes davon eine, z. B. gemäss der Variante a) erhältliche, Verbindung der Formel I, worin $R_4$ Wasserstoff ist, oder ein Salz davon, vorzugsweise in Gegenwart einer Base, mit einer Verbindung der Formel

$$X\text{-}R_4 \qquad (III),$$

die bekannt ist oder in Analogie zu entsprechenden bekannten Verbindungen hergestellt werden kann und worin $R_4$ eine der für die Formel I angegebenen Bedeutungen mit Ausnahme von Wasserstoff hat und X für eine Abgangsgruppe steht, oder gegebenenfalls einem Salz davon umsetzt oder
c) zur Herstellung einer Verbindung der Formel I, worin $R_4$ Methyl oder eine Gruppe $R_2CH_2$- ist, oder eines Salzes davon eine Verbindung der Formel

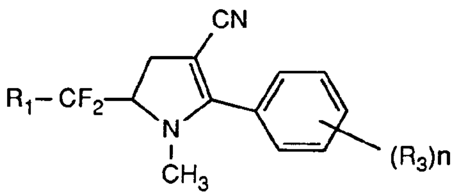
(VII),

worin $R_1$, $R_3$ und n die für die Formel I angegebenen Bedeutungen haben, oder ein Salz davon mit einem Halogenierungsmittel, gegebenenfalls in Gegenwart einer Base, gegebenenfalls in Gegenwart eines Radikalstarters, umsetzt und jeweils, wenn erwünscht, eine verfahrensgemäss oder auf andere Weise erhältliche Verbindung der Formel I, in freier Form oder in Salzform, in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

2. Verfahren gemäss Anspruch 1 zur Herstellung einer Verbindung der Formel I in freier Form.

3. Verfahren gemäss Anspruch 2 zur Herstellung einer Verbindung der Formel I, worin $R_1$ Perfluor-$C_1$-$C_6$-alkyl bedeutet.

4. Verfahren gemäss Anspruch 2 zur Herstellung einer Verbindung der Formel I, worin $R_2$ Chlor oder Brom ist.

5. Verfahren gemäss Anspruch 2 zur Herstellung einer Verbindung der Formel I, worin n die Zahl 1, 2, 3, 4 oder 5, wobei, wenn n grösser als 1 ist, die Reste $R_3$ gleich oder verschieden sind, und $R_3$ Halogen, Halogen-$C_1$-$C_4$-akyl, Halogen-$C_1$-$C_4$-alkoxy, Cyano, $C_1$-$C_4$-Akyl, $C_1$-$C_4$-Alkoxy, $C_1$-$C_4$-Alkylthio oder $C_1$-$C_4$-Alkansulfonyl und/oder zwei an benachbarte C-Atome des Phenylrings gebundene Substituenten $R_3$ gemeinsam -O-C(Halogen)$_2$-O-, insbesondere -O-$CF_2$-O-, oder -CH=CH-CH=CH- bedeuten.

6. Verfahren gemäss Anspruch 2 zur Herstellung einer Verbindung der Formel I, worin $R_4$ Wasserstoff, $C_1$-$C_4$-Akyl, $C_3$-$C_4$-Alkenyl, Halogen-$C_3$-$C_4$-alkenyl, $C_3$-$C_4$-Alkinyl, Cyano, $C_1$-$C_4$-Alkylthio, $C_1$-$C_4$-Alkansulfinyl, $C_1$-$C_4$-Alkansulfonyl, Halogen-$C_1$-$C_4$-alkansulfonyl, Di-$C_1$-$C_4$-alkylaminosulfonyl, Di-$C_1$-$C_4$-alkylaminocarbonyl oder eine durch einen oder mehrere Substituenten, ausgewählt aus der Gruppe, bestehend aus Halogen, $C_1$-$C_4$-Alkoxy, Hydroxy, $C_1$-$C_4$-Alkylthio, $C_2$-$C_6$-Alkoxyalkoxy, $C_1$-$C_4$-Alkansulfinyl, $C_1$-$C_4$-Alkansulfonyl, $C_1$-$C_4$-Akylcarbonyl, $C_1$-$C_4$-Alkoxycarbonyl, $C_1$-$C_4$-Alkylcarbonyloxy, Cyano, $C_2$-$C_4$-Alkenylcarbonyloxy, Phenyl, Phenoxy, Phenylthio, Benzyloxy, Benzoyloxy, Benzoyl, Phenylsulfinyl und Phenylsulfonyl, wobei die in diesen Phenyl-, Phenoxy-, Phenylthio-, Benzyloxy-, Benzoyloxy-, Benzoyl-, Phenylsulfinyl- und Phenylsulfonyl-Substituenten enthaltenen Phenylkerne jeweils durch Halogen, $C_1$-$C_4$-Akyl, $C_1$-$C_4$-Alkoxy, Halogen-$C_1$-$C_4$-alkyl, Halogen-$C_1$-$C_4$-alkoxy, $C_1$-$C_4$-Alkylthio, $C_1$-$C_4$-Alkansulfonyl, Halogen-$C_1$-$C_4$-alkylthio, Halogen-$C_1$ -$C_4$-alkansulfinyl, Halogen-$C_1$-$C_4$-alkansulfonyl, Cyano oder Nitro substituiert sein können, substituierte $C_1$-$C_4$-Akylgruppe bedeutet.

7. Verfahren gemäss Anspruch 2 zur Herstellung einer Verbindung der Formel I, worin $R_4$ Wasserstoff, $C_1$-$C_4$-Alkyl, $C_3$-$C_4$-Alkinyl, $C_1$-$C_4$-Alkansulfonyl, Di-$C_1$-$C_4$-alkylaminosulfonyl oder eine durch $C_1$-$C_4$-Alkoxy, $C_1$-$C_4$-Alkylthio, $C_2$-$C_6$-Alkoxyalkoxy, $C_1$-$C_4$-Alkansulfonyl, $C_1$-$C_4$-Alkylcarbonyl, $C_1$-$C_4$-Alkoxycarbonyl,

Cyano, Monohalogenphenyl oder Phenoxy substituierte $C_1$-$C_4$-Akylgruppe bedeutet.

8. Verfahren gemäss Anspruch 2 zur Herstellung einer Verbindung der Formel I, worin $R_1$ Perfluor-$C_1$-$C_6$-alkyl, $R_2$ Chlor oder Brom, $R_3$ Halogen, Halogen-$C_1$-$C_4$-alkyl, Halogen-$C_1$-$C_4$-alkoxy, Cyano, $C_1$-$C_4$-Akyl, $C_1$-$C_4$-Alkoxy, $C_1$-$C_4$-Alkyltio oder $C_1$-$C_4$-Alkansulfonyl, $R_4$ Wasserstoff, $C_1$-$C_4$-Akyl oder $C_2$-$C_6$-Alkoxyalkyl und n die Zahl eins, zwei oder drei bedeuten, wobei n die Zahl eins, zwei, drei, vier oder fünf bedeuten kann, wenn alle Reste $R_3$ gleichzeitig für Halogen stehen.

9. Verfahren gemäss Anspruch 8 zur Herstellung einer Verbindung der Formel I, worin $R_1$ Trifluormethyl oder Pentafluorethyl, $R_2$ Brom, $R_3$ Fluor, Chlor, Trifluormethyl, Fluor-$C_1$ -$C_3$-alkoxy, Methylthio, Cyano oder $C_1$-$C_3$-Alkoxy, $R_4$ Wasserstoff, Methyl, Methoxymethyl oder Ethoxymethyl und n die Zahl eins, zwei oder drei bedeuten.

10. Verfahren gemäss Anspruch 8 zur Herstellung einer Verbindung der Formel I, worin $R_1$ Trifluormethyl, $R_2$ Chlor oder Brom, $R_3$ Fluor, Chlor, Trifluormethyl, Fluor-$C_1$ -$C_3$-alkoxy, Methylthio, Cyano oder $C_1$-$C_3$-Alkoxy, $R_4$ Wasserstoff, Methyl, Methoxymethyl oder Ethoxymethyl und n die Zahl eins, zwei oder drei bedeuten.

11. Verfahren gemäss Anspruch 9 zur Herstellung einer Verbindung der Formel I, worin n die Zahl 1, $R_1$ Trifluormethyl oder Pentafluorethyl, $R_2$ Brom, $R_3$ Trifluormethyl, Trifluormethoxy oder Chlor und $R_4$ Wasserstoff oder Ethoxymethyl bedeuten.

12. Verfahren gemäss Anspruch 2 zur Herstellung einer Verbindung der Formel I, ausgewählt aus der Gruppe, bestehend aus den Verbindungen
(a) 4-Brom-2-(4-chlorphenyl)-3-cyano-5-heptafluorpropyl-pyrrol,
(b) 4-Brom-2-(4-chlorphenyl)-3-cyano-1-ethoxymethyl-5-pentafluorethyl-pyrrol,
(c) 4-Brom-2-(4-chlorphenyl)-3-cyano-1-ethoxymethyl-5-heptafluorpropyl-pyrrol,
(d) 4-Chlor-2-(4-chlorphenyl)-3-cyano-5-heptafluorpropyl-pyrrol,
(e) 4-Chlor-2-(4-chlorphenyl)-3-cyano-1-ethoxymethyl-5-pentafluorethyl-pyrrol,
(f) 4-Chlor-2-(4-chlorphenyl)-3-cyano-1-ethoxymethyl-5-heptafluorpropyl-pyrrol,
(g) 4-Brom-3-cyano-5-pentafluorethyl-2-(4-trifluormethylphenyl)-pyrrol,
(h) 4-Chlor-3-cyano-5-pentafluorethyl-2-(4-trifluormethylphenyl)-pyrrol,
(i) 4-Brom-3-cyano-1-ethoxymethyl-5-pentafluorethyl-2-(4-trifluormethylphenyl)-pyrrol,
(j) 4-Chlor-3-cyano-1-ethoxymethyl-5-pentafluorethyl-2-(4-trifluormethylphenyl)-pyrrol,
(k) 4-Brom-3-cyano-5-pentafluorethyl-2-(4-trifluormethoxyphenyl)-pyrrol,
(l) 4-Chlor-3-cyano-5-pentafluorethyl-2-(4-trifluormethoxyphenyl)-pyrrol,
(m) 4-Brom-3-cyano-1-ethoxymethyl-5-pentafluorethyl-2-(4-trifluormethoxyphenyl)pyrrol und
(n) 4-Chlor-3-cyano-1-ethoxymethyl-5-pentafluorethyl-2-(4-trifluormethoxyphenyl)-pyrrol.

13. Schädlingsbekämpfungsmittel, dadurch gekennzeichnet, dass es mindestens eine Verbindung, erhältlich gemäss Anspruch 1, der Formel I, in freier Form oder in agrochemisch verwendbarer Salzform, als Wirkstoff und gegebenenfalls mindestens einen Hilfsstoff enthält.

14. Mittel gemäss Anspruch 13 zur Bekämpfung von Insekten, Vertretern der Ordnung Akarina und/oder phytopathogenen Pilzen.

15. Verfahren zur Herstellung eines mindestens einen Hilfsstoff enthaltenden Mittels gemäss Anspruch 12 oder 13, dadurch gekennzeichnet, dass man den Wirkstoff mit dem (den) Hilfsstoff(en) innig vermischt und/oder vermahlt.

16. Verwendung einer Verbindung, erhältlich gemäss Anspruch 1, der Formel I, in freier Form oder in agrochemisch verwendbarer Salzform, zur Herstellung eines Mittels gemäss Anspruch 13 oder 14.

17. Verwendung eines Mittels gemäss Anspruch 13 oder 14 zur Bekämpfung von Schädlingen.

18. Verwendung gemäss Anspruch 17 zur Bekämpfung von Insekten, Vertretern der Ordnung Akarina und/ oder phytopathogenen Pilzen.

19. Verwendung gemäss Anspruch 17 oder 18 zum Schutz von pflanzlichem Vermehrungsgut.

**20.** Verfahren zur Bekämpfung von Schädlingen, dadurch gekennzeichnet, dass man ein Mittel gemäss Anspruch 13 oder 14 auf die Schädlinge und/oder ihren Lebensraum appliziert.

**21.** Verfahren gemäss Anspruch 20 zur Bekämpfung von Insekten, Vertretern der Ordnung Akarina und/oder phytopathogenen Pilzen.

**22.** Verfahren gemäss Anspruch 20 oder 21 zum Schutz von pflanzlichem Vermehrungsgut, dadurch gekennzeichnet, dass man das Vermehrungsgut oder den Ort der Ausbringung des Vermehrungsguts behandelt.

**23.** Pflanzliches Vermehrungsgut, behandelt gemäss dem in Anspruch 22 beschriebenen Verfahren.

**24.** Verfahren zur Herstellung einer Verbindung der Formel

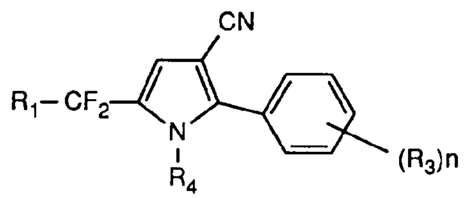

(II),

worin $R_1$, $R_3$, $R_4$ und n die für die Formel I angegebenen Bedeutungen haben, in freier Form oder in Salzform, dadurch gekennzeichnet, dass man

d) zur Herstellung einer Verbindung der Formel II, worin $R_4$ Wasserstoff ist, oder eines Salzes davon eine Verbindung der Formel

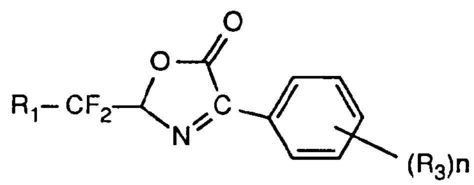

(IV),

worin $R_1$, $R_3$ und n die für die Formel I angegebenen Bedeutungen haben, oder ein Salz davon, vorzugsweise in einem hochsiedenden polaren Lösungsmittel, wie Nitromethan, und bei der Rückflusstemperatur des Reaktionsgemisches, mit 2-Chloracrylnitril umsetzt oder

e) zur Herstellung einer Verbindung der Formel II, worin $R_4$ von Wasserstoff verschieden ist, oder eines Salzes davon eine, z. B. gemäss der Variante d) erhältliche, Verbindung der Formel II, worin $R_4$ Wasserstoff ist, oder ein Salz davon, vorzugsweise in Gegenwart einer Base, z. B. von der unter der Variante a) angegebenen Art, mit einer Verbindung der Formel

$$X\text{-}R_4 \qquad \text{(III)},$$

die bekannt ist oder in Analogie zu entsprechenden bekannten Verbindungen hergestellt werden kann und worin $R_4$ eine der für die Formel I angegebenen Bedeutungen mit Ausnahme von Wasserstoff hat und X für eine Abgangsgruppe, z. B. von der unter der Variante b) angegebenen Art, steht, oder gegebenenfalls einem Salz davon umsetzt und jeweils, wenn erwünscht, eine verfahrensgemäss oder auf andere Weise erhältliche Verbindung der Formel II, in freier Form oder in Salzform, in eine andere Verbindung der Formel II überführt, ein verfahrensgemäss erhältliches Gemisch von Isomeren auftrennt und das gewünschte Isomere isoliert und/oder eine verfahrensgemäss erhältliche freie Verbindung der Formel II in ein Salz oder ein verfahrensgemäss erhältliches Salz einer Verbindung der Formel II in die freie Verbindung der Formel II oder in ein anderes Salz überführt.

**25.** Verwendung einer Verbindung, erhältlich gemäss Anspruch 24, der Formel II, in freier Form oder in Salzform, zur Herstellung einer Verbindung der Formel I.

**26.** Verfahren zur Herstellung einer Verbindung der Formel

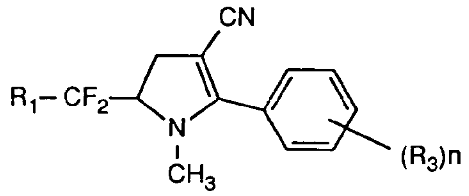

(VII),

worin $R_1$, $R_3$ und n die für die Formel I angegebenen Bedeutungen haben, in freier Form oder in Salzform, dadurch gekennzeichnet, dass man

f) eine Verbindung der Formel

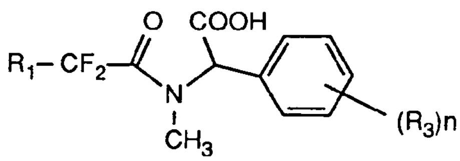

(VIII),

worin $R_1$, $R_3$ und n die für die Formel I angegebenen Bedeutungen haben, oder ein Salz davon, vorzugsweise in einem hochsiedenden polaren Lösungsmittel, wie Acetonitril, in Gegenwart eines Säureanhydrids, wie in Gegenwart von Acetanhydrid, und bei der Rückflusstemperatur des Reaktionsgemisches, mit Acrylnitril umsetzt und, wenn erwünscht, eine verfahrensgemäss oder auf andere Weise erhältliche Verbindung der Formel VII, in freier Form oder in Salzform, in eine andere Verbindung der Formel VII überführt, ein verfahrensgemäss erhältliches Gemisch von Isomeren auftrennt und das gewünschte Isomere isoliert und/oder eine verfahrensgemäss erhältliche freie Verbindung der Formel VII in ein Salz oder ein verfahrensgemäss erhältliches Salz einer Verbindung der Formel VII in die freie Verbindung der Formel VII oder in ein anderes Salz überführt.

**27.** Verwendung einer Verbindung, erhältlich gemäss Anspruch 26, der Formel VII, in freier Form oder in Salzform, zur Herstellung einer Verbindung der Formel I.

Europäisches Patentamt

# EUROPÄISCHER RECHERCHENBERICHT

Nummer der Anmeldung

EP 92 81 0635

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-0 372 982 (RHONE-POULENC) 13. Juni 1990 * das ganze Dokument * --- | 1-30 | C07D207/34 C07D207/22 A01N43/36 C07D207/48 |
| Y | EP-A-0 088 743 (MONSANTO) 14. September 1983 * das ganze Dokument * --- | 1-30 | |
| P,Y | EP-A-0 492 171 (AMERICAN CYANAMID) 1. Juli 1992 * das ganze Dokument * --- | 1-30 | |
| D,Y | EP-A-0 426 948 (AMERICAN CYANAMID) 15. Mai 1991 * das ganze Dokument * --- | 1-30 | |
| D,Y | EP-A-0 347 488 (AMERICAN CYANAMID) 27. Dezember 1989 * das ganze Dokument * --- | 1-30 | |
| D,Y | EP-A-0 372 263 (AMERICAN CYANAMID) 13. Juni 1990 * das ganze Dokument * --- | 1-30 | RECHERCHIERTE SACHGEBIETE (Int. Cl.5 ) |
| D,Y | EP-A-0 434 940 (AMERICAN CYANAMID) 3. Juli 1991 * das ganze Dokument * ----- | 1-30 | C07D A01N |

Der vorliegende Recherchenbericht wurde für alle Patentansprüche erstellt

| Recherchenort | Abschlußdatum der Recherche | Prüfer |
|---|---|---|
| DEN HAAG | 21 OKTOBER 1992 | Bernd Kissler |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)